# EUROPEAN PATENT APPLICATION

(11) **EP 2 301 573 A1**
(43) Date of publication of application: **30.03.2011**
(21) Application number: 10181660.1
(22) Date of filing: 01.10.2003
(51) Int. Cl.: A61K 39/12, A61K 48/00, A61K 31/00

(54) **Anti-cancer and anti-infectious disease compositions and methods for using same**

(30) Priority: 01.10.2002 US 416660 P; 01.10.2002 US 414649 P
(62) Divisional of application: 03773107.2
(71) Applicant: Novartis Vaccines and Diagnostics, Inc., Emeryville, CA 94608 (US)
(72) Inventor: Kavanaugh, Michael W., Emeryville, CA 94608 (US); Sloane, David L., 94608 Emeryville, CA (US); Mackichan, Mary Lee, 94608 Emeryville, CA (US)
(74) Representative: UEXKÜLL & STOLBERG

(57) **Abstract**

The present invention provides methods and compositions for treating and/or preventing cancer in a subject via administration of a non-pathogenic virus. The present invention also provides methods and compositions for treating and/or preventing infectious diseases in a subject via administration of a non-pathogenic virus.

## Description

### FIELD OF THE INVENTION

The present invention generally relates to cancer therapy. More particularly, the present invention relates to the use of non-pathogenic viruses as effective anti-cancer agents.

### BACKGROUND OF THE INVENTION

### Table of Abbreviation

| | | |
|---|---|---|
| A549 | - | human lung epithelial tumor cell line |
| AcNPV | - | *Autographa californica* nucleopolyhedrosis virus |
| BMDC | - | bone marrow-derived dendritic cells |
| BV422 | - | recombinant baculovirus expressing CCL21 |
| BV762 | - | recombinant baculovirus expressing Raf |
| CCL21 | - | C-C motif Ligand 21 Chemokine; Secondary Lymphoid-Tissue Chemokine |
| CD86 | - | marker for dendritic cell maturation |
| CR | - | complete response |
| CTL | - | cytotoxic T lysis |
| DC | - | dendritic cell |
| FACS | - | fluorescence activated cell sorting |
| GM-CSF | - | Granulocyte-Macrophage Colony-Stimulating Factor |
| GV | - | granulosis virus |
| HIV | - | human immunodeficiency virus |
| i.t. | - | intratumorally |
| mCCL21 | - | mouse CCL21 |
| MHC | - | major histocompatibility complex |
| MHC II | - | MHC class II |
| MLA-DR | - | MHC class I antigen |
| MOl | - | multiplicity of infection |
| NPV | - | nucleopolyhedrosis virus |
| PBMCs | - | peripheral blood mononuclear cells |
| PFU | - | plaque-forming unit |
| qd | - | Quaque Die (given daily) |
| rhCCL21 | - | recombinant human GCL21 |
| s.c. | - | subcutaneously |
| Sf(Sf9) | - | *Spodoptera frugiperda* |
| Tn (Tn5) | - | *Trichoplusia ni* |
| UV | - | ultraviolet |
| VLP | - | virus-like particle |

### Background

Modulation of immune response has become an important anti-cancer strategy. A significant effort in the design of cancer vaccines and immunotherapies has focused on the identification of antigens that are selectively present in tumor cells. Unique tumor immunogenicity has permitted induction of tumor-specific immune responses using vaccines that include tumor-specific antigens, or genes expressing tumor-specific antigens. Vaccination approaches have also included adoptive cellular methods, whereby antigen-presenting cells are modified to present tumor-associated antigens. Additional immunological strategies for cancer treatment include administration of cytokines and chemokines, which have therapeutic potential as adjuvants or treatments in anti-cancer therapies based on their ability to expand and recruit immune effector cells. See *e.g.,* Homey et al. (2002) Nat Rev Immunol 2:175-84; Parmiani et al. (2002) J Natl Cancer Inst 94:805-18; Bronte (2001) Curr Gene Ther 1:53-100; and Fehniger et al. (2002) Cytokine Growth Factor Rev 13:169-83.

Notwithstanding the above-noted advances, the success of immunological approaches has been limited by: (1) tumor-specific antigenicity, such that therapies are limited to particular cancer types; (2) poor antigen presentation by tumor cells; and (3) and the ability of tumor cells to produce immune inhibitory factors to thereby escape immune sunveillance. Thus, there exists a long-felt and continuing need in the art for effective and broadly applicable cancer therapies. To meet this need, the present invention provides novel immunostimulatory methods for cancer treatment and prevention.

### SUMMARY OF THE INVENTION

The present invention provides methods of inducing an immune response in an animal comprising administering to the animal an amount of a composition comprising an inactive non-pathogenic virus effective to induce an immune response in the animal.

The present invention further provides methods of causing cell death in a cell comprising administering a composition comprising an amount of a non-pathogenic virus to the cell effective to cause cell death in the cell.

Further, the present invention provides methods of eliciting a CTL response in an animal comprising administering a composition comprising an amount of a non-pathogenic virus to the animal effective to elicit a CTL response in the animal, wherein the non-pathogenic virus is an insect-specific virus.

The present invention provides methods of inhibiting tumor growth in an animal comprising administering to the animal an amount of a composition comprising a non-pathogenic insect-specific virus effective to inhibit tumor growth in the animal.

The present invention also provides methods of effecting cancer remission in an animal comprising administering to the animal an amount of a composition comprising a non-pathogenic virus effective to effect cancer remission.

The present invention further provides methods of inhibiting cancer metastasis in an animal comprising administering to the animal an amount of a composition comprising a non-pathogenic virus effective to inhibit cancer metastasis in the animal.

The present invention provides methods of imparting resistance to cancer re-challenge in an animal comprising administering a composition comprising a non-pathogenic virus to the animal.

Further, the present invention provides methods of inhibiting a non-neoplastic proliferative disorder in an animal comprising administering a composition comprising a non-pathogenic virus to the animal.

The present invention also provides methods of inhibiting hyperplasia or metaplasia in an animal comprising administering to the animal an amount of a composition comprising a non-pathogenic virus effective to inhibit hyperplasia in the animal.

The present invention provides methods of inhibiting one or more symptoms of cancer in an individual in need thereof comprising administering to the individual an amount of a composition comprising a non-pathogenic virus effective to inhibit one or more symptoms of cancer in the individual.

Further, the present invention provides methods of protecting an animal from an infectious disease comprising administering to the animal an amount of a composition comprising an inactive non-pathogenic virus effective to protect the animal from an infectious disease.

The present invention also provides methods of inhibiting an infectious disease in an animal comprising administering to the animal an amount of a composition comprising a non-pathogenic virus effective to inhibit the infectious disease in the animal.

The present invention provides methods of causing cell death in a population of cells comprising contacting a composition comprising an amount of a non-pathogenic virus to a portion of the population of cells effective to cause cell death in the population of cells.

The present invention provides methods of treating a disease in a subject in need thereof comprising inactivating a non-pathogenic virus, wherein the nonpathogenic virus is inactivated by adding trioxalen to the non-pathogenic virus at a concentration between about 5-10 µg/ml and illuminating the non-pathogenic virus with UV at about 365 nm and about 6W for about 15 minutes, formulating the inactivated non-pathogenic virus into a pharmaceutical composition; and administering the pharmaceutical composition to the subject.

The present invention also provides methods of predicting *in vivo* anti-tumor activity of a compound comprising contacting the compound with tumor cells and peripheral blood mononuclear cells; and measuring cell death of the tumor cells. Compounds that cause cell death of contacted tumor cells are predicted to be active *in vivo.*

Further, the present invention provides methods of preventing cancer in an individual comprising administering to the individual an amount of a composition comprising a non-pathogenic virus effective to prevent cancer in the individual.

Also, the present invention provides compositions comprising a non-pathogenic virus and peripheral blood mononuclear cells (PBMCs).

The present invention further provides compositions comprising a non-pathogenic virus inactivated by at least two different methods.

The present invention also provides compositions comprising a non-pathogenic virus, the non-pathogenic virus inactivated using two or more methods selected from the group consisting of genetic inactivation, chemical inactivation, photochemical inactivation, UV-light inactivation., heat inactivation, or radiological inactivation, and at least one PBMC.

The present invention provides processes for preparing an anti-cancer or anti-infectious disease composition comprising a non-pathogenic virus. The processes comprise exposing the virus to a first inactivator effective to inactivate an active virus, exposing the virus to a second inactivator effective to inactivate an active virus, combining the virus with one or more pharmaceutically acceptable carriers or excipients, and confirming inactivity of the virus in an *in vitro* assay.

The present invention further provides pharmaceutical compositions comprising an inactivated non-pathogenic virus and at least one antigen, wherein the antigen is distinct from the inactivated non-pathogenic virus, and at least one adjuvant.

Further, the present invention provides immunostimulating compositions comprising an adjuvant composition. The adjuvant composition comprises an inactivated non-pathogenic virus, at least one antigen. The antigen is distinct from the adjuvant composition, and further the immunostimulating composition is capable of increasing the immune response to the antigen.

The methods disclosed herein are directed to the treatment of human subjects, however, they can be used for the treatment of any mammal in need thereof, In some embodiments, cancers and non-neoplastic disorders that can be treated or prevented include, but are not limited to, those of the lung, breast, and skin. In some embodiments infectious diseases that can be treated or prevented include viral infections.

The present invention further provides that a non-pathogenic virus used in accordance with the anti-cancer methods disclosed herein can comprise a live virus, an inactivated virus, a viral particle, a virosome, a Viral Like Particle, a viral occlusion body, or a viral component In some embodiments, viral components include, for example, peptide, proteins, nucleic acids, lipids, carbohydrates, and combinations thereof, in some embodiments, the viral component is gp64.

In some embodiments, the non-pathogenic virus is an insect-specific virus. In some embodiments, the insect-specific virus is a virus of the family of Baculoviridae. For example, a non-pathogenic virus of the invention can comprise a nucleopolyhedrosis virus or a granulosis virus. In some embodiments a non-pathogenic virus is Autographa californica nuclepolyhedrosis virus.

In some embodiments, for the treatment of tumors, a non-pathogenic virus and optionally an antigen and/or adjuvant are administered to a mammalian subject intratumorally and/or peritumorally. In some embodiments, for the treatment of non-neoplastic proliferative disorders, a non-pathogenic virus and optionally an antigen and/or adjuvant are administered to a mammalian subject intralesionally and/or perilesionally. A therapeutic regimen can include multiple administrations of a non-pathogenic virus, optionally in combination with other anti-cancer therapies.

### BRIEF DESCRIPTION OF THE DRAWINGS

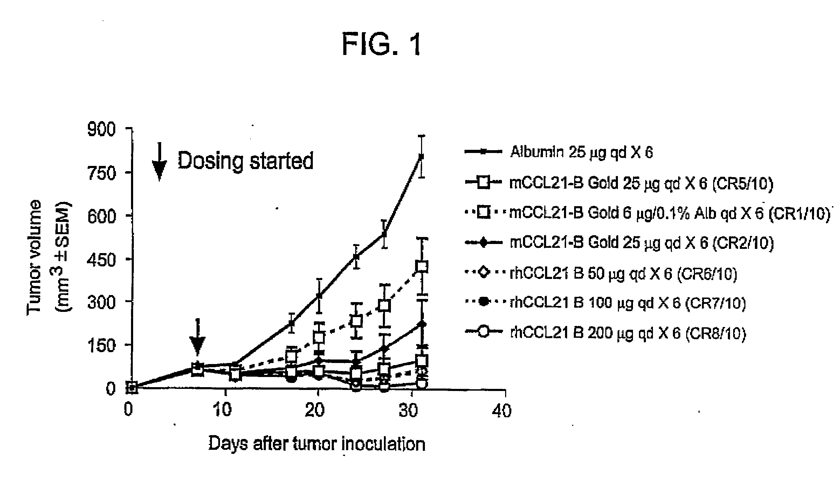

Figure 1 depicts a line graph, which shows that baculovirus-expressed CCL21 inhibits tumor growth and induces complete tumor remission in a 3LL mouse model of lung cancer. As described in Example 2, CCL21 administration included 6 intratumoral injections of CCL21 at the concentrations indicated. Increasing the dose per injection increased tumor inhibition and the frequency of complete responses. One way ANCVA analysis was performed using data collected on day 24. Albumin compared with treated groups, P<0.001; 25 µg mCCL21 compared with 6 µg/Alb mCCL21, P<0.01; 25 µg mCCL21 compared with 25 µg rhCCL21, P>0.05. mCCL21, mouse CCL21; rhCCL21, recombinant human CCL21; B Gold, recombinant mouse CCL21 expressed from baculovirus, which was used as a reference lot ("Gold Standard"); Alb, albumin; qd, Quaque Die (given daily), CR, complete response.

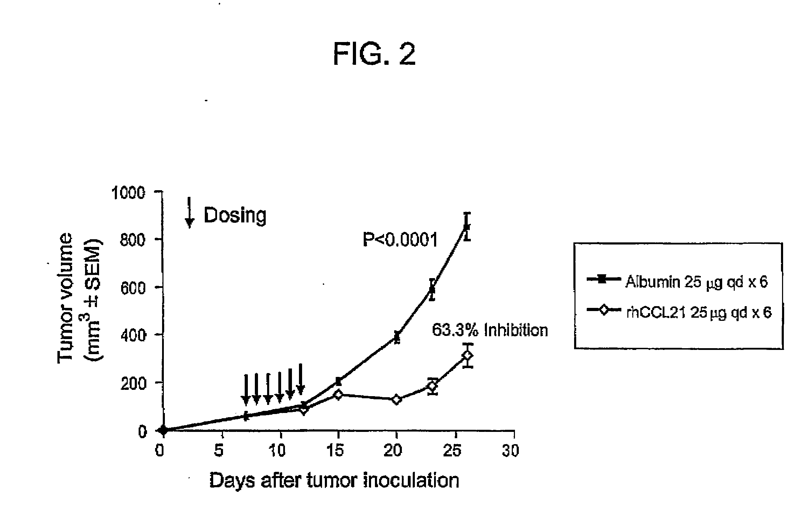

Figure 2 depicts a line graph, which depicts inhibition of tumor growth in a 4T1 mouse model of breast cancer following administration of baculovirus-expressed CCL21, as described in Example 3. rhCCL21, recombinant human CCL21; qd, Quaque Die (given daily).

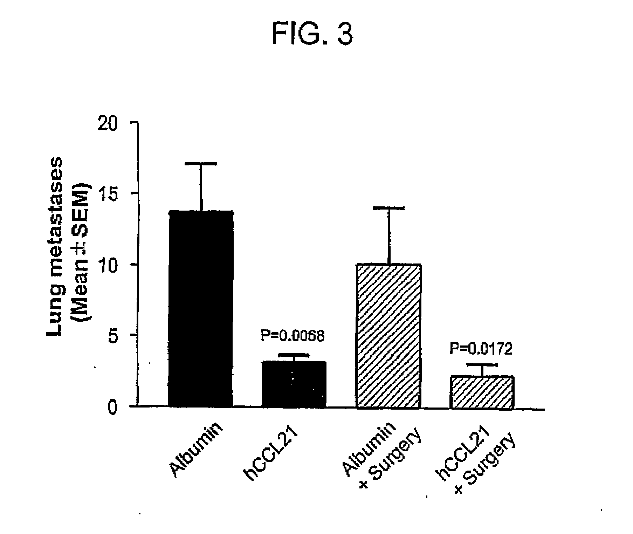

Figure 3 depicts a bar graph, which depicts inhibition of spontaneous 4T1 lung metastasis following administration of baculovirus-expressed CCL21, as described in Example 6. Solid bars, animals that did not undergo surgical resection of the tumor; hatched bars, animals in which tumors were surgically resected (Surg) 1 day after the last dose of CCL21.

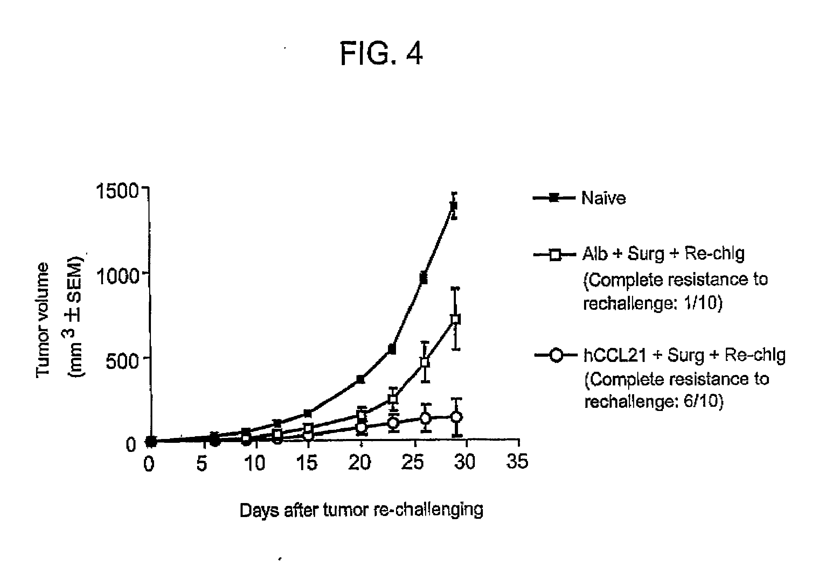

Figure 4 depicts a line graph, which shows that administration of baculovirus-expressed CCL21 imparts resistance to tumor re-challenge, as described in Example 5. Briefly, 4T1 tumors were established in Balb/c mice, and a subset of host mice were treated via intratumoral administration of baculovirus-expressed CCL21. One day after the last dose of CCL21. tumors were surgically resected. One day after tumor resection, mice were re-challenged with s.c. injection of 4T1 cells at a site contralateral to the original tumor. Naive, mice that had not previously hosted a tumor and which did not receive CCL21 treatment; Alb + Surg + Re-chlg, mice that had previously hosted a 4T1 tumor and that received albumin treatment; hCCL21 + Surg + Re-chlg, mice that had previously hosted a 4T1 tumor and that received CCL21 treatment, In the Alb + Surg + Re-chlg group, 1 of 10 mice showed complete resistance to tumor growth. In the hCCL21 + Surg + Re-chlg group, 6 of 10 mice showed complete resistance to tumor growth.

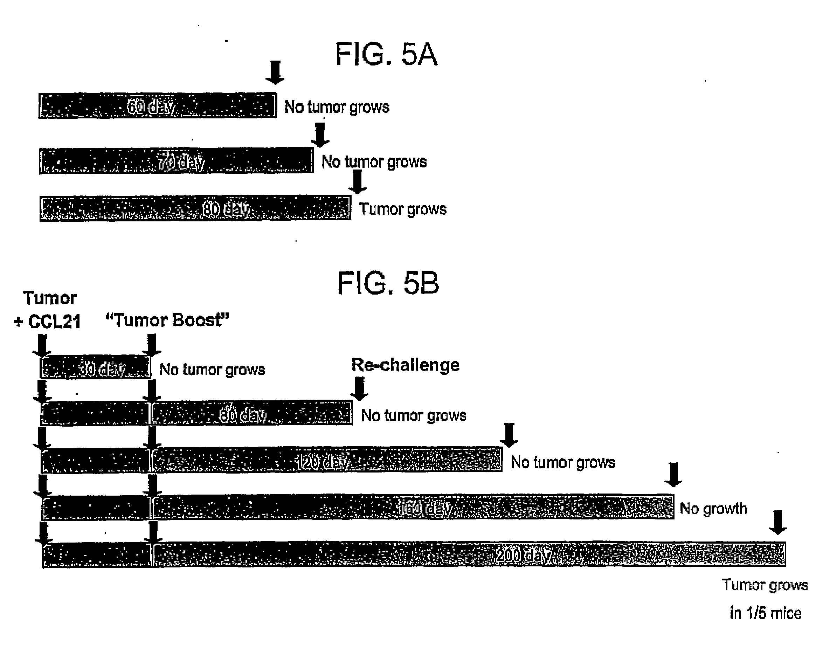

Figures 5A-5B demonstrate baculovirus-induced resistance to tumor re-challenge. In the 3LL tumor model, animals that have been successfully treated with baculovirus-derived CCL21 are resistant to re-challenge with the same tumor for prolonged periods. Figure 5A summarizes experiments with animals that had a complete remission of tumors after treatment with baculovirus-derived mouse recombinant CCL21 (see e.g., Figure 1), and were re-challenged in the opposite flank with the same tumor at 60, 70 and 80 days following the last dose of baculovirus-derived CCL21. Animals were resistant to re-challenge for at least 70 days. Figure 5B summarizes experiments with animals that received an inoculation 30 days following completion of baculovirus CCL21 treatment and induction of complete remission ("tumor boost"), and that were re-challenged in the opposite flank with the same tumor at 80, 120, 160 and 200 days following the last dose of baculavirus-derived CCL21. These results demonstrate that resistance to tumor re-challenge can be extended to at least 200 days by using a tumor boost

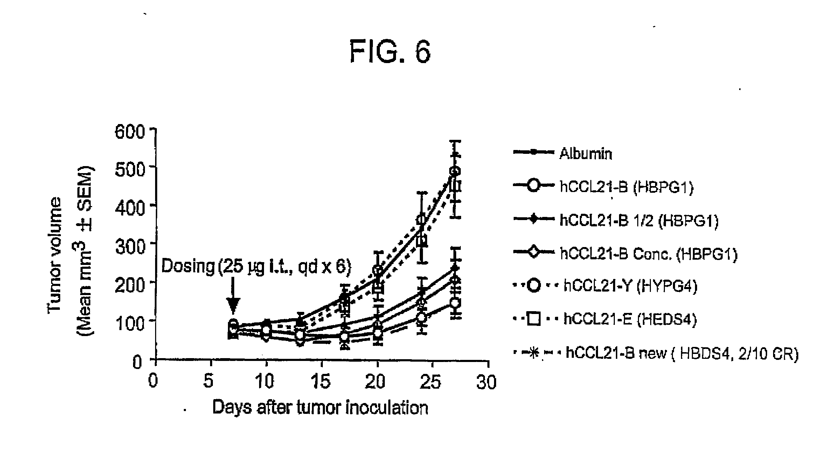

Figure 6 depicts a line graph, which shows that CCL21 produced in yeast or E. coli does not result in tumor remission in a 3LL tumor model of lung cancer. hCCL21-B (HBPG1), recombinant human CCL21 expressed in baculovirus, lot# HBPG1; hCCL21-B ½ (HBPG1), recombinant human CCL21 expressed in baculovirus, lot# HBPG1, diluted to one-half of the concentration of hCCL21 (HBPG1); hCCL21-B conc. (HBPG1), recombinant human CCL21 expressed in baculovirus, lot# HBPG1 derived from a concentrated (10 mg/ml) solution; hCCL21-Y (HYPG4), recombinant human CCL21 expressed in yeast, lot# HYPG4; hCCL21-E (HEDS4), recombinant human CCL21 expressed in E.coli, lot# HEDS4; qd, Quaque Die (given daily). Inhibition of tumor growth in mice treated with albumin when compared to mice treated with hCCL21-B or with hCCL21-B new, P <0.01; inhibition of tumor growth in mice treated with hCCL21-B conc. or with hCCL21-P ½, P < 0.05.

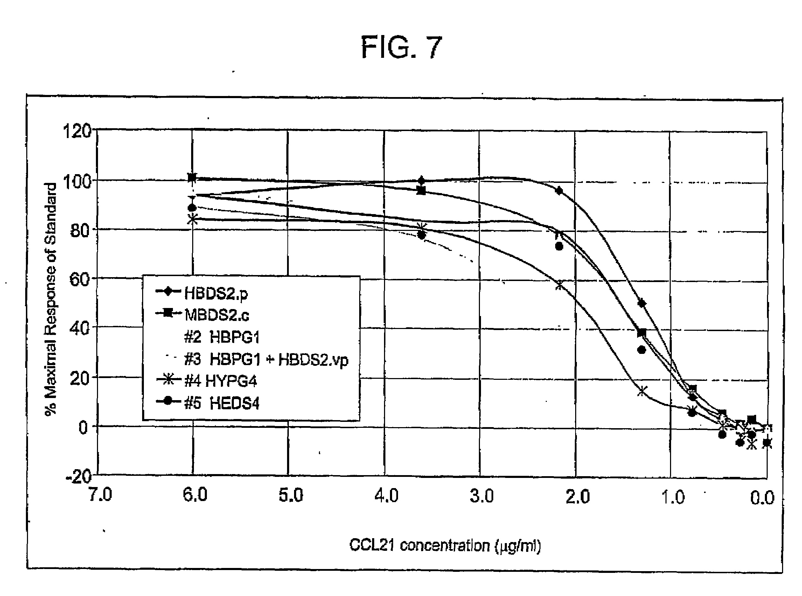

Figure 7 depicts a line graph, which shows *in vitro* chemotaxis activity of baculovirus-expressed-CCL21 preparations that are inactive *in vivo.* The chemotaxis assay can be performed essentially as described in PCT International Publication No. WO .00/38706. *In vivo* activity was assessed as described in Examples 2-6. HBDS2.p, recombinant human CCL21 derived from baculovirus, lot# HBDS2.p; MBDS2.c, recombinant mouse CCL21 derived from baculovirus, lot# MBDS2.c; HBPG1, recombinant human CCL21 derived from baculovirus, lot# HBPG1; HBPG1 + HBDS2.vp, HBPG1 mixed with an equimolar ratio of HBDS2 that had been treated with vinyl pyridine; HYPG4, recombinant human CCL21 derived from yeast, lot# HYPG4; HEDS4, recombinant human CCL21 derived from E. coli, lot# HEDS4.

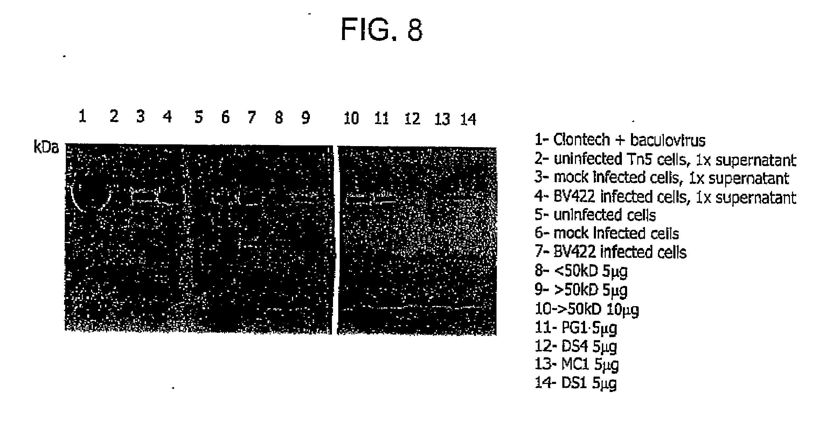

Figure 8 depicts a Western blot that was prepared using the indicated samples and then probed with an anti-gp64 antibody. Lane 1, purified baculovirus; Lane 2, conditioned media from uninfected Tn5 cell culture; Lane 3, conditioned media from Tn5 cells infected with wild type baculovirus; Lane 4, conditioned media from Tn5 cell culture infected with BV422 encoding recombinant human CCL21; Lane 5, uninfected Tn5 cell pellet; Lane 6, cell pellet from Tn5 culture infected with wild type baculovirus; Lane 7, cell pellet from Tn5 culture infected with BV422; Lane 8, human recombinant CCL21 derived from baculovirus, lot# HBPG1, filtrate after removing contaminants >50 kDa; Lane 9, retentate from sample in Lane 8 containing contaminants >50 kDa (5 µg of protein); Lane 10, retentate from sample in Lane 8 containing contaminants >50 kDa (10 µg of protein); Lane 11, 5 µg of unfiltered recombinant human baculovirus-derived CCL21, lot# HBPG1; Lane 12, 5 µg of unfiltered recombinant human baculovirus-derived GCL21, lot# HBDS4; Lane 13, 5 µg of unfiltered recombinant human baculovirus-derived CCL21, lot# PBMC1; Lane 14, 5 µg of unfiltered recombinant human baculovirus-derived CCL21, lot# HBDS1.

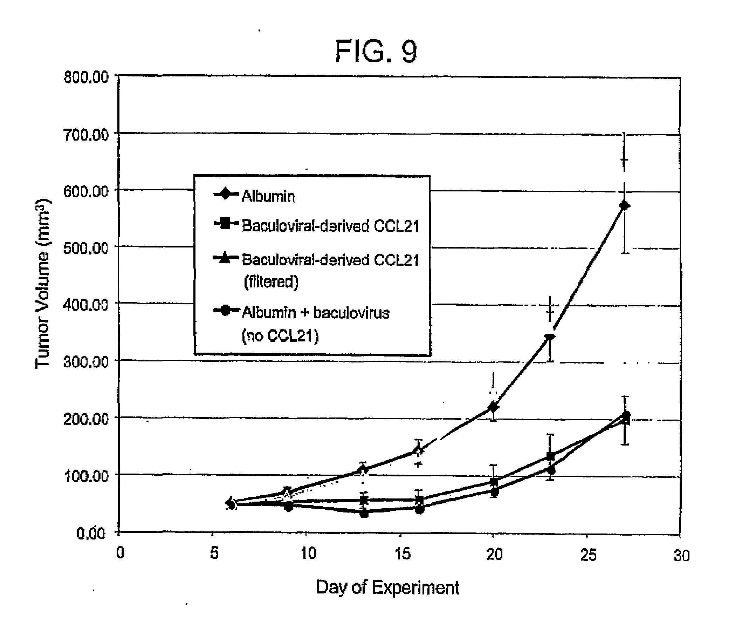

Figure 9 depicts a line graph, which shows that the anti-tumor activity of baculovirus-expressed CCL21 is removed by filtering to remove high molecular weight contaminants from the preparation. Purified baculovirus, a contaminant of the CCL21 preparations (Figure 8), inhibits tumor growth as effectively as baculovirus-expressed CCL21 preparations.

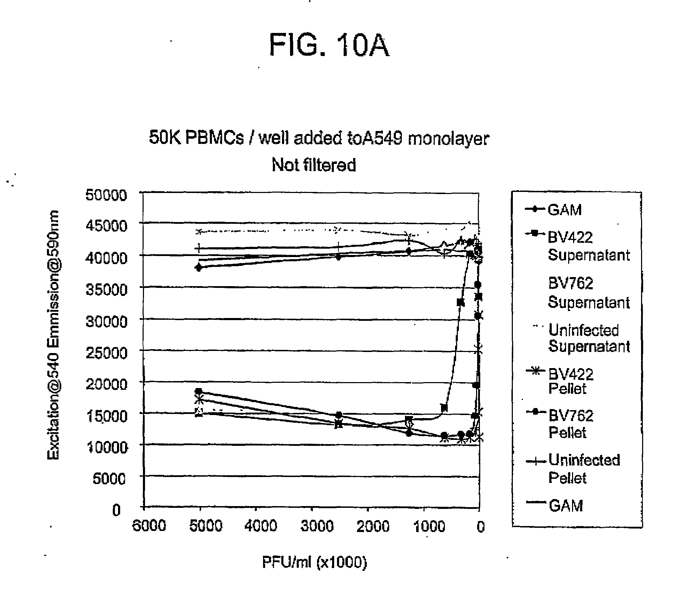

Figure 10A depicts a line graph that shows PBMC-mediated toxicity of tumor cells *in vitro* when exposed to the indicated compositions, as described in Example 8. The cell pellet samples induced a greater cytotoxic response than the supernatant samples. Cells infected with either CCL21-expressing baculovirus or the control baculovirus, BV762, showed significant cytotoxicity. GAM, growth assay media that has not been conditioned by cell culture (control); BV422, baculovirus expressing CCL21; BV762, baculovirus expressing Raf.

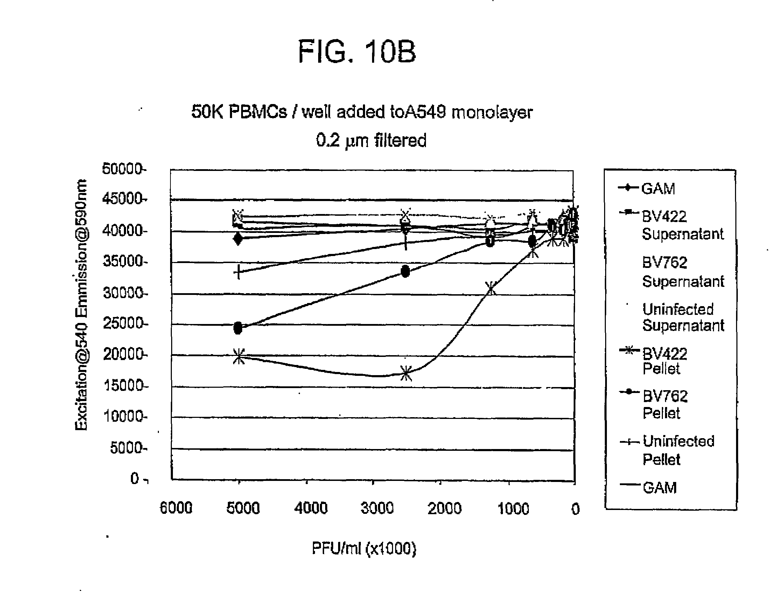

Figure 10B depicts a line graph that shows PBMC-mediated toxicity of tumor cells *in vitro* when exposed to the indicated compositions following filtering through a 0.2 µm filter, as described in Example 8. Removal of high molecular weight substances by filtering significantly reduced cytotoxicity. The cell pellet samples showed residual low to moderate cytotoxicity.

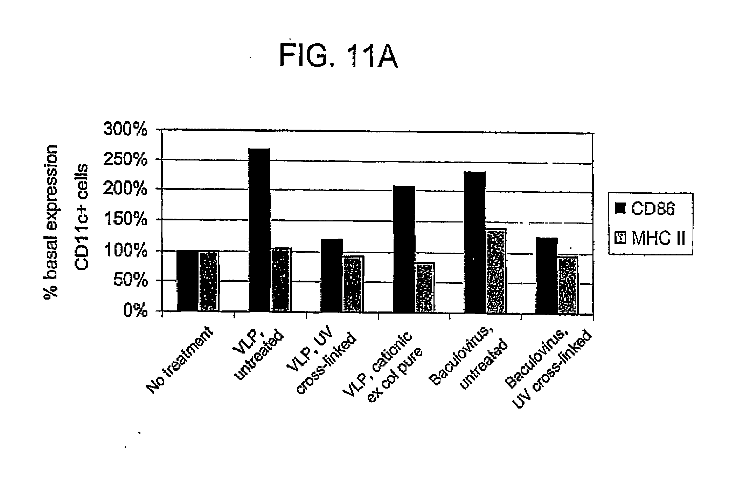

Figure 11A depicts a bar graph, which shows changes in dendritic cell expression of CD86 and MHC II in response to the indicated stimuli. Mouse bone marrow-derived dendritic cells were prepared and analyzed as described in Example 9. Vaccinia virus expressing HIV gag protein (VLP) was used as a positive control. Similar to VLP, live baculovirus induced CD86 and MHC II expression, which is indicative of DC maturation. Black bars, CD86 expression; gray bars, MHC II expression.

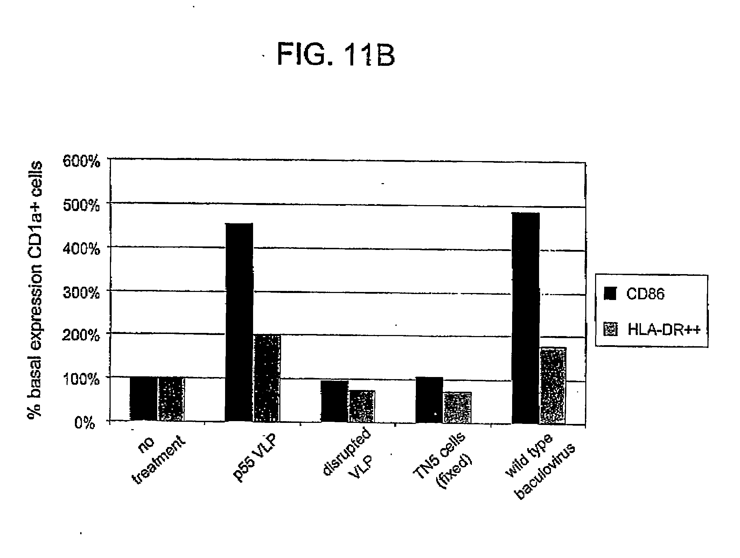

Figure 11B depicts a bar graph, which shows changes in dendritic cell expression of CD86 and MHC II in response to the indicated stimuli. Human monocyte-derived dendritic cells were prepared and analyzed as described in Example 9. Vaccinia virus expressing the HIV gag protein (p55 VLP) was used as a positive control. Similar to p55 VLP, live baculovirus induced DC maturation. Black bars, CD86 expression; gray bars, HLA-DR expression.

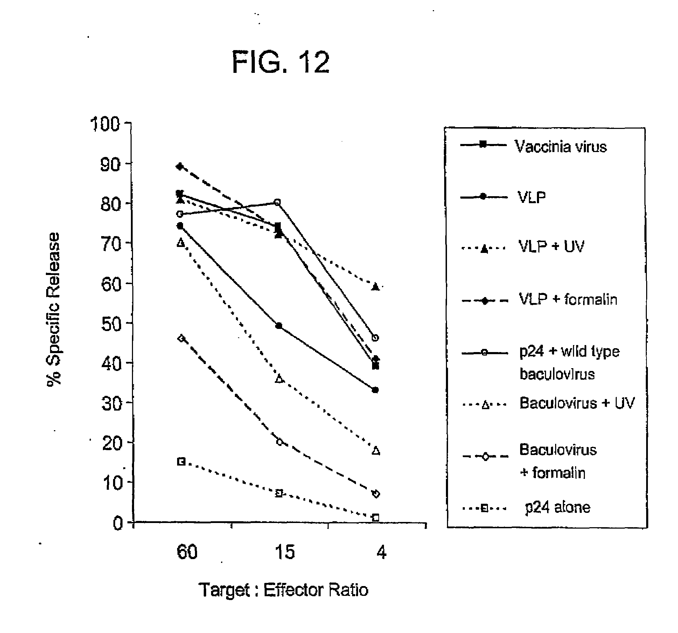

Figure 12 depicts the results of a chromium release assay, which was performed as described in Example 10. Vaccinia virus expressing HIV gag protein (VLP) was used as a positive control. Similar to VLP, live baculovirus acts as a potent adjuvant to induce cytotoxic T cell lysis.

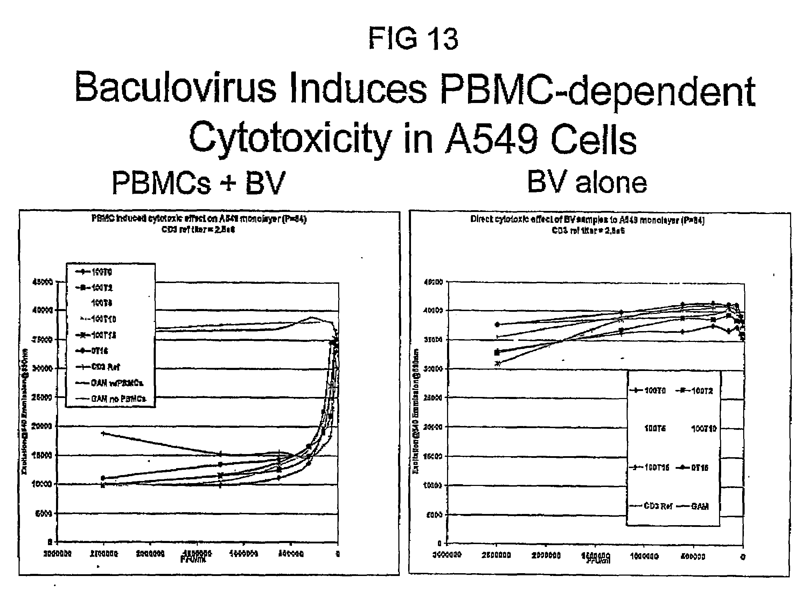

Figure 13 depicts PBMC-mediated toxicity of tumor cells *in vitro* when exposed to the indicated compositions, as described in Example 8.

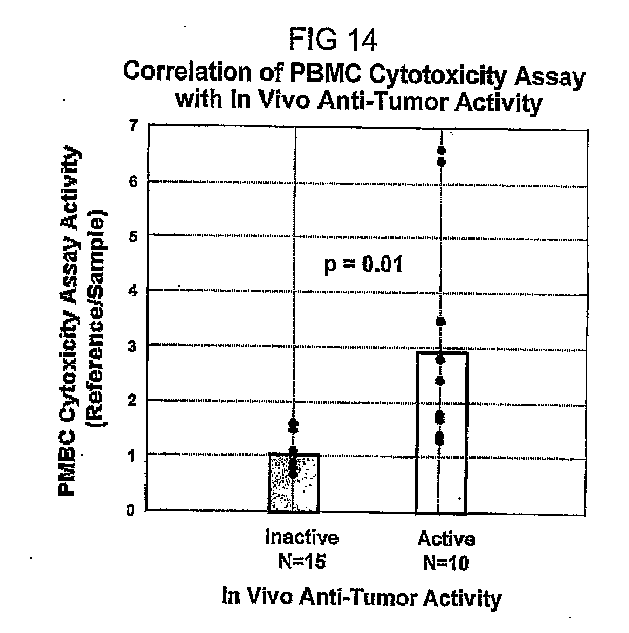

Figure 14 depicts the correlation of PBMC Cytotoxicity Assay with *in vivo* anti-tumor activity.

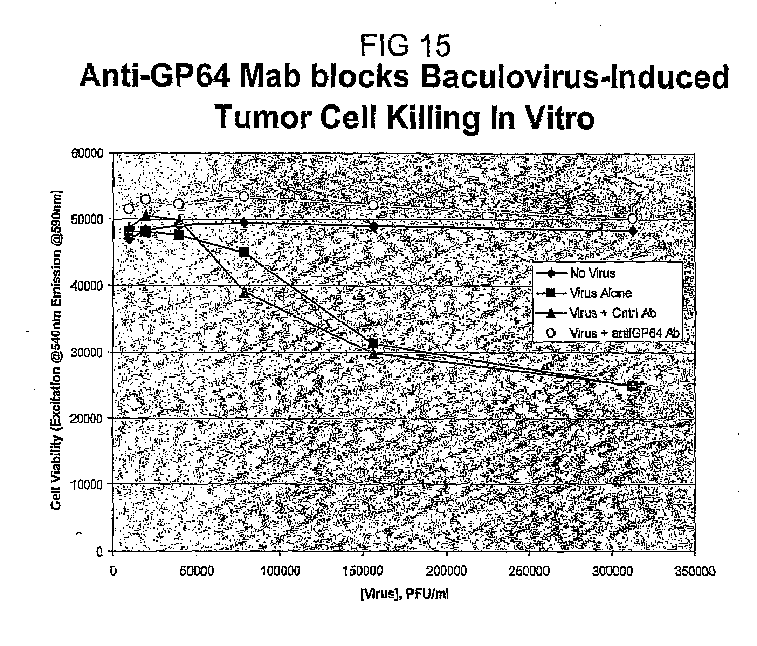

Figure 15 depicts the blockage of Baculovirus Tumor Cell Killing by anti-gp64 Monoclonal Antibodies. See Example 13.

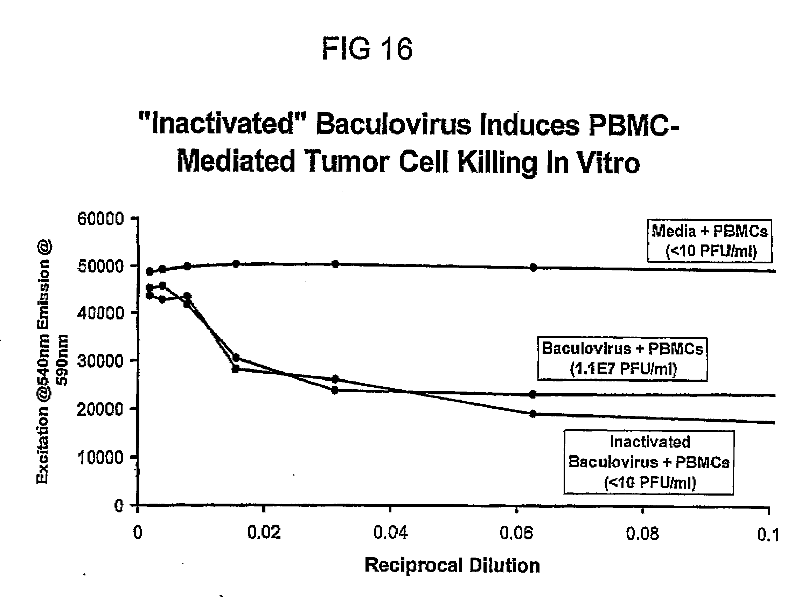

Figure 16 depicts PBMC-Mediated Tumor Cell Killing *in vitro* induced by inactivated baculovirus, See Example 14.

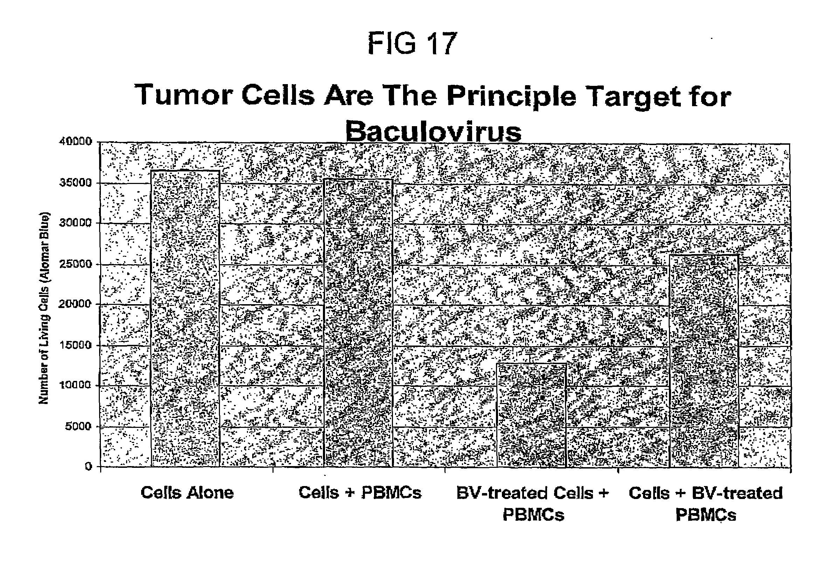

Figure 17 demonstrates that tumor cells are the primary targets for baculovirus. See Example 15.

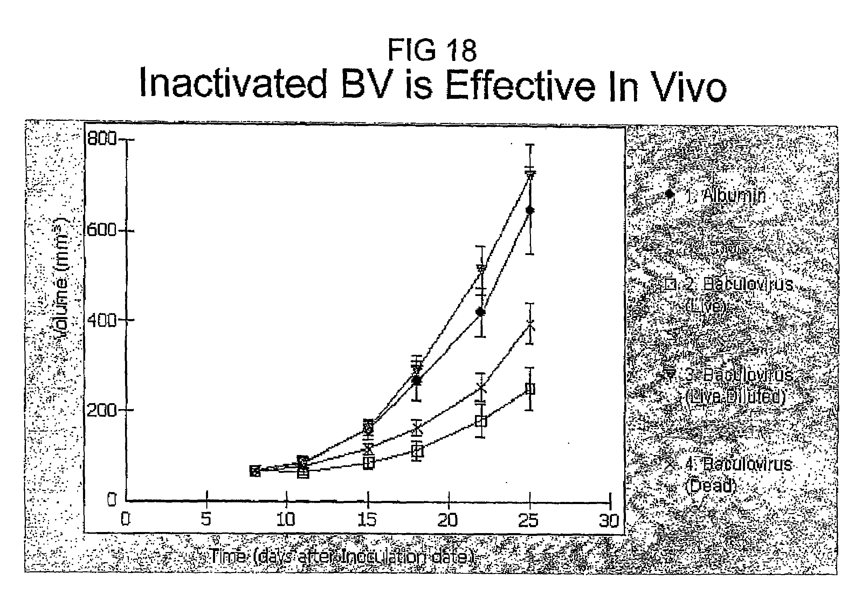

Figure 18 depicts tumor growth inhibition in an animal model of lung cancer using baculovirus. See Example 16.

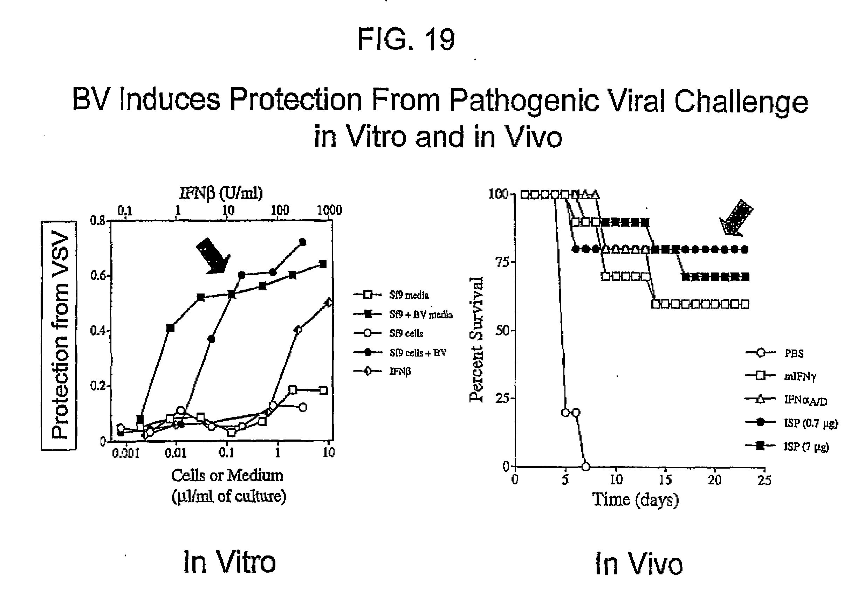

Figure 19 depicts baculovirus-induced protection from pathogenic viral challenge *in vivo* and *in vitro.* Cells were challenged *in vivo* and *in vitro* with Vesicular Stomatitis Virus (VSV). See Example 17.

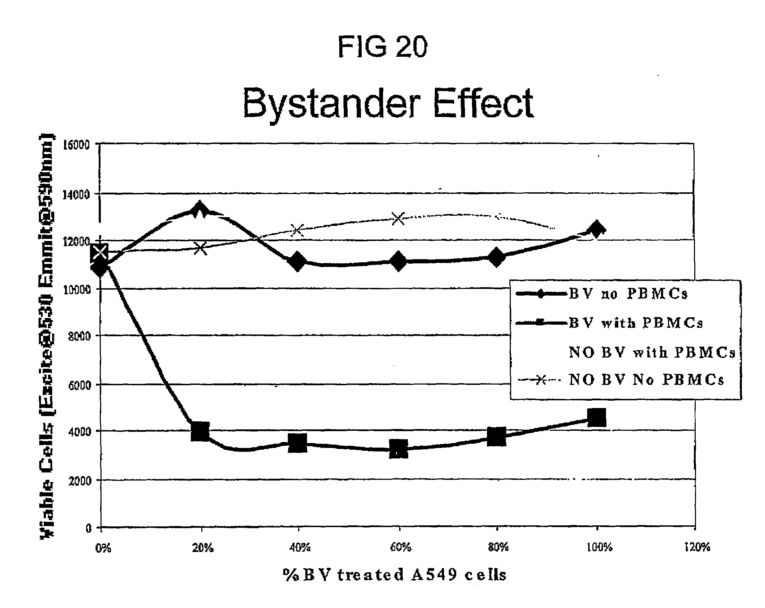

Figure 20 depicts the bystander effect. Maximal killing effect is achieved after exposing as few as 20% of a population of tumor cells to baculovirus.

### DETAILED DESCRIPTION OF THE INVENTION

### A1. Anti-tumor Activity of Non-Pathogenic Viruses

Immunological approaches to cancer treatment include the use of chemokines as therapeutic agents. Chemokines are a family of homologous proteins whose functions include: (a) mediating lymphocyte migration and activation; (2) regulating angiogenesis; (c) and maintaining immune homeostasis and secondary lymphoid organ architecture. See Baggiolini et al. (1997) Annu Rev Immunol 15:675-705; Jung & Littman (1999) Curr Opin lmmunol 11:319-25; and Homey et al. (2002) Nat Rev lmmunol 2:175-84.

In the course of developing cancer immunotherapies that employ Secondary Lymphoid Tissue Chemokine (referred to herein as "CCL21:" also known in the art as SLC, Exodus-2, and 6C-kine), as described in Examples 1-6, the inventors of the subject disclosure came to the surprising discovery that non-pathogenic viruses are potent anti-tumor agents. See Example 7.

Thus, the present invention provides, *inter alia,* methods for treating a subject in need of anti-cancer therapy, including inhibition of cancer growth, inhibition of cancer metastasis, and cancer resistance, via administration of a non-pathogenic virus to the subject. Significantly, the cancer immunotherapies disclosed herein do not rely on identification of tumor-specific antigens. Rather, administration of non-pathogenic viruses is broadly applicable and is efficacious in multiple tumor types. See Examples 2-6.

While the inventors do not wish to be bound to a particular mode of operation, the inventors suggest that the anti-cancer activity of non-pathogenic viruses is attributable, at least in part, to their immunostimulatory properties. For example, baculovirus activates dendritic cell maturation and cytolytic T cell (CTL) responses both *in vitro* and *in vivo.* See Examples 9-10. See also Gronowski et al. (1999) J Virol. 73:9944-51.

The term "virus," as used herein to describe an effective anti-tumor composition, encompasses live virus, inactivated virus, virus particles, viral occlusion bodies, virosomes, Viral Like Particles, viral components, and combinations thereof.

Virosomes and Virus Like Particles (VLPs) generally contain one or more proteins from a non-pathogenic virus optionally combined or formulated with a phospholipid. In some embodiments virosomes and VLPs are non-replicating and do not contain any of the native viral genome, The viral proteins may be recombinantly produced or isolated from whole viruses. VLPs are discussed further in WO 03/024480, WO 03/024481, Niikura et al., (Chimeric Recombinant Hepatitis E Virus-Like Particles as an Oral Vaccine Vehicle Presenting Foreign Epitopes", Virology (2002) 293:273 - 280); Lenz et al., (Papillomarivurs-Like Particles Induce Acute Activation of Dendritic Cells, Joumal of Immunology (2001) 5246 - 5355); Pinto, et al., (Cellular immune Responses to Human Papillomavirus (HPV)-16 L1 Healthy Volunteers Immunized with Recombinant HPV-16 L1 1 Virus-Like Particles", Journal of Infectious Diseases (2003) 188:327 - 338); and Gerber et al., (Human Papillomavirus Virus-Like Particles Are Efficient Oral lmmunagens when Coadministered with Escherichia coli Heat-Labile Entertoxin Mutant R192G or CpG), Journal of Virology (2001) 75(10):4752 - 4760. Virosomes are discussed further in, for example, Gluck et al., (New Technology Platforms in the Development of Vaccines for the Future, Vaccine (2002) 20:B10-B16.)

The term "live virus" refers to a virus whose infectivity is similar or identical to a native virus. In particular, a live virus can infect its native host cells.

The term "inactivated virus" refers to a virus that is incapable of replication in a native host cell, as described further herein below. For example, a non-pathogenic virus, which is incapable of replication in a mammalian host cell, is similarly incapable of replication in it native host cell upon being inactivated. Inactivated viruses can be used to minimize safety concerns regarding administration of live viruses. An "inactivator" is the agent utilized to inactivate the virus.

The term ".virus particle" refers to a virus that has been constructed, or modified from its native form, whereby it is unable to replicate in naturally occurring host cells. Methods for preparing virus particles are known in the art. The structural and functional integrity of virus-like particles can be assessed by electron microscopy, immunogenicity analyses, and standard plaque assays. For example, Hamakubo, et. al WO 02/06305 discuss generation of enucleated baculoviral-like particles.

U.S. Patent No. 5,760,383 discloses methods for preparing baculovirus particles using a marker-rescue system. The method employs a genetically modified baculovirus, which lacks a gene essential for viral replication (e.g., gp64), and which is propagated in cells that complement the genetic deficiency.

The term "viral occlusion body" refers to a structure comprising a multiplicity of viral particles embedded within a virus-encoded proteinaceous crystal. Upon dissolution of the protein crystal, the multiplicity of viral particles is released, and each viral particle is capable of subsequent infection of a host cell.

Production of viruses, and in particular baculoviruses, is accomplished using techniques well known in the art. Cloned cell lines are provided in a culture medium *in vitro,* inoculated with virus, and incubated for a sufficient time and under conditions effective to allow viral production. Culture conditions, including cell density, multiplicity of infection, time, temperature, media, etc. are not critical and can be readily determined by a practitioner skilled in the art.

Representative methods for baculovirus production are described in Example 1, which employ Spodoptera frugiperda (Sf) cells. Additional representative host cells and amplification methods are described in U.S. Patent Nos. 5,405,770 (Heliothis subflexa cell line) and 6,379,958 (Spodoptera frugiperda cell lines, which show improved baculovirus productian).

Following incubation, the viral agents so produced are recovered by techniques conventional in the art, including polyethylene glycol (PEG) precipitation, ultracentrifugation, and chromatographic purification, such as use of an ion exchange resin, size exclusion chromatography, affinity chromatography, or combinations thereof. See U.S. Patent Application Publication No. 2002/0015945 (chromatographic purification); U.S. Patent No. 6,194,192 (viral adsorption to sulfated-fucose-containing polyseccharide(s)).

The term "viral component," as used herein, refers to a molecule that is derived from a non-pathogenic virus and that retains anti tumor and/or anti-infectious disease activity of the parent live virus. In some embodiments, a viral component comprises anti-tumor activity that is similar in magnitude and specificity of response when compared to that elicited by the parent live virus from which it was derived. The term "viral component" encompasses any biological component of a virus including, for example, one or more of a protein, a peptide, a nucleic acid, a lipid, a carbohydrate, any other bioactive molecule of a virus, and combinations thereof. In some embodiments, the viral component is gp64.

For example, a viral component can comprise a viral capsid protein or a DNA-associated protein of the viral nucleoprotein core. Representative baculoviral capsid proteins are described by Pearson et al. (1988) Virology 167:407-13; by Summers & Smith (1978) Virology 84:390402; by Thiem & Miller (1989) J Virol 63:2008-18; and by Vialard & Richardson (1993) J Virol 67:5859-66. Representative baculoviral DNA-associated proteins are described by Tweeten et al. (1980) J Virol 33:866-876; by Wilson et al. (1987) J Virol 61:661-6; and by Rohrmann (1992) J Gen Virol 73 ( Pt 4):749-61.

A viral component can also comprise proteins and carbohydrates found in viral occlusion bodies, including the occlusion body matrix and the calyx outer layer found in mature occlusion bodies. Representative baculovirus occlusion body proteins include polyhedron and calyx.

Following a review of the disclosure herein, which provides that non-pathogenic viruses have potent anti-tumor activity and/or anti-infectious disease activity, a skilled artisan could readily identify, purify or otherwise prepare, and administer viral components to recapitulate the anti-tumor activity and/or anti-infectious disease of the parent live virus. For example, as one approach, U.S. Patent No. 6,001,806 discloses biochemical methods for fractionating baculovirus-infected insect cells, and then using the eluate fractions in assays to identify a glycoprotein that mimics the anti-viral activity previously.recognized in the parent live virus.

In addition, viral proteins and nucleic acids are readily prepared using recombinant methods known in the art and can be similarly tested for anti-cancer and/or anti-infectious disease activity. For example, viral nucleic acids can be cloned, synthesized, altered, mutagenized, or combinations thereof. Standard recombinant DNA and molecular cloning techniques used to isolate nucleic acids can be found, for example, in Sambrook et al. (eds.) (1989) Molecular Cloning: A Laboratory Manual. Cold Spring Harbor Laboratory Press, Cold Spring Harbor, New York; Silhavy et al. (1984) Experiments with Gene Fusions. Cold Spring Harbor Laboratory Press, Cold Spring Harbor, New York; Glover & Hames (1995) DNA Cloning: A Practical Approach, 2nd ed. IRL Press at Oxford University Press, Oxford/New York; and Ausubel (ed.) (1995) Short Protocols in Molecular Biology, 3rd ed. Wiley, New York. Recombinantly produced polypeptides can also be purified and characterized using a variety of standard techniques that are known to the skilled artisan. See e.g., Schröder & Lübke (1965) The Peptides. Academic Press, New York; Schneider & Eberle (1993) peptides, 1992: Proceedings of the Twenty-Second European Peptide Symposium, September 13-19. 1992, Interiaken, Switzenand. Escom, Leiden; Bodanszky (1993) Principles of Peptide Synthesis, 2nd rev. ed. Springer-Verlag, Berlin; New York; and Ausubel (ed.) (1995) Short Protocols in Molecular Biology, 3rd ed. Wiley, New York.

In particular, the complete sequence of AcNPV is known (Kool and Vlak, 1993), and thus a systematic analysis of all AcNPV proteins can be readily performed using known methods for recombinant expression in combination with assays for anti-tumor activity.

To facilitate identification of active viral components, the present invention further provides an *in vitro* assay that can be used to screen candidate viral components. See Example 8. The assay involves induction of cytotoxicity by peripheral blood mononuclear cells (PBMCs). As disclosed herein, a non-pathogenic virus can induce cytotoxicity of tumor cells by PBMCs, and this activity correlates with anti-tumor activity observed upon *in vivo* administration. Candidate viral components include, for example, biochemical fractions of a non-pathogenic virus, purified or recombinantly produced viral proteins, purified or synthesized nucleic acids, virosomes, Virus Like Particles, and the like.

The term "non-pathogenic," as used herein to describe a virus, refers to a virus that is not infectious in a mammalian host to be treated with the virus, and in some embodiments, a non-pathogenic virus is not infectious in any mammalian host In some embodiments, a non-pathogenic virus is not infectious in a human host For the sake of convenience, unless otherwise indicated, the term "non-pathogenic" virus includes inactivated virus, virus particles, viral occlusion bodies, virosomes, Viral Like Particles, viral components, and combinations thereof.

The term "infectious" generally refers to a property of being deleterious to a host cell or organism, for example by expression of genes that are deleterious to the host cell or organism and/or by replication in the host. Consistent with this definition, non-pathogenicity does not preclude entry into a mammalian cell, wherein such entry does not compromise the health of the host cell or organism. However, in some embodiments, a non-infectious virus does not enter into a mammalian cell.

A non-pathogenic virus, for example baculovirus, can also be transcriptionally silent in mammalian host cells. Thus, in some embodiments, a non-pathogenic virus can be a type of virus that is specifically excluded from current gene therapy methods, as heterologous genes are also not expressed.

Examples of non-pathogenic viruses include, but are not limited to, insect specific viruses, amphibian-specific viruses, and plant-specific viruses. Representative viruses useful in the methods disclosed herein include viruses of the family Baculoviridae (e.g., nucleopolyhedroviruses (NPV) such as Autographa californica NPV, and granuloviruses (GV) such as Trichoplusia ni GV), Polydnaviridae (e.g., ichnoviruses such as Campoletis sonorensis virus, and bracoviruses such as Cotesia molanoscela virus), Ascoviruses, Tetraviridae, and Nodaviridae (e.g., nodaviruses such as Nodamura virus and Flock House Virus). A number of non-pathogenic viruses useful in the present invention are found in insects. See Fields et al., eds. (1996) Virology, Lippincott-Rave Publishers, Philadelphia, Pennsylvania.

The term "infectious disease" refers to an agent (e.g. virus, fungi or bacteria) that is deleterious to its host. In some embodiments the agent is deleterious to a human host. An "anti-infectious disease" treatment refers to a treatment that prevents, ameliorates or eradicates the infectious disease and/or its disease-causing agent.

Examples of infectious diseases include without limitation, HIV, West Nile virus, hepatitis A, B, C, small pox, tuberculosis, Vesicular Stomatitis Virus (VSV), Respiratory Syncytial Virus (RSV), human papilloma virus (HPV), SARS, influenza, Ebola, viral meningitis, herpes, anthrax, lyme disease, and E. Coli., among others.

in some embodiments, a non-pathogenic virus comprises a baculovirus. As described in the Examples below, the present invention provides that baculovirus is a potent inhibitor of tumor growth and can promote complete tumor remission. The present invention further provides that baculovirus can be used to inhibit tumor metastasis and to promote resistance to tumor re-challonge, as described in Examples 5-6.

As used herein, the term "tumor re-challenge" refers to animal whose cancer or tumor has been treated or removed and then is exposed to a new tumor. In accordance with the definition provided herein above, the term "baculovirus" encompasses baculovirus particles and baculovirus components.

The host specificity of baculovirus has been thoroughly studied. Although baculovirus is known to infect over 30 species of Lepidoptera, it is not thought to be competent to replicate in other insect cells or in any of the over 35 mammalian cell lines studied. See Tjia et al. (1983) Virology 125:107-17; Volkman & Goldsmith (1983) Appl Environ Microbiol 45:1085-1093; and McIntosh & Shamy (1980) Intervirology 13:331-41. Baculovirus does, however, enter mammalian cells and viral DNA can be detected in the host cell nucleus, See Groner et al. (1984) lntervirology 27:203-9; Tjia et al. (1983) Virology 125:107-17; and Volkman & Goldsmith (1983)Appi Environ Microbiol 45:1085-93.

The term "non-pathogenic" further encompasses viruses, which are pathogenic in their native form, and which have been modified to be non-pathogenic. Such modification can include genetic modification (e.g., disruption of a gene that is essential for viral replication, as described herein above for the baculovirus gp64 gene; and/or disruption of a viral promoter to render it transcriptionally inactive in the host species). For example, the species-specific pathogenicity of baculovirus is due in part to silence of the baculovirus promoter in species other than Lepidoptera. When a heterologous promoter is inserted into baculovirus genome, the modified virus becomes capable of gene expression in non-Lepidopteran cell lines, including various mammalian cell lines. See Boyce & Bucher (1996) Proc Natl Acad Sci USA 93:2348-52; Carbonell et al. (1985) J Virol 56:153-60; Carbonell & Miller (1987) Appl Environ Microbiol 53:1412-7; and Hofmann et al. (1995) Proc Natl Acad Sci USA 92:10099-103. A viral promoter that is initially active in mammalian cells could be similarly modified to the opposite result, whereby it is no longer pathogenic in mammalian species. Methods for site-specific mutagenesis to create base pair changes, deletions, or small insertions are also known in the art, for example as described in the references noted herein above.

Modified viruses, as well as unmodified viruses that are suspected to be non-pathogenic, can be readily assayed for non-pathagenicity using methods for determining viral infectivity and replication known in the art. Representative methods can be found, for example, in Tjia et al. (1983) Virology 125:107-17; Volkman & Goldsmith (1983) Appl Environ Microbiol 45:1085-93; McIntosh& Shamy (1980) Intervirology 13:331-41; and U.S. Patent No. 6,248,514, among other places.

The present invention also provides non-pathogenic viruses having anti-cancer activity and/or anti-infectious disease activity and/or adjuvant activity, including live viruses, inactive viruses, viral particles, virosomes, VLPs, viral occlusion bodies, and viral components. Also provided are methods for selecting a non-pathogenic virus useful in the therapeutic methods described herein.

To select a non-pathogenic virus having anti-cancer activity, candidate non-pathogenic viruses can be tested using an *in vitro* or *in vivo* assay of tumor cytolysis, as described in Example 8, and/or an *in vivo* or *in vivo* model of anti-cancer activity, for example as described in the Examples. In some embodiments, an *in vitro* assay can be used as an initial screen, and then viruses that are active *in vitro* can be subsequently tested in relevant animal models to assess anti-cancer activity,

To select a non-pathogenic virus having adjuvant activity, candidate non-pathogenic viruses can be tested for ability to enhance immunogenicity of an antigen. Immunogenicity can be determined by, for example, detecting T cell-mediated responses. Representative methods for measuring T cell responses include *in vitro* cytotoxicity assays or *in vivo* delayed-type hypersensitivity assays. In some embodiments, for example, CCL21 in combination with a non-pathogenic virus can induce *in vitro* cytotoxicity of tumor cells by PBMCs, and this activity correlates with anti-tumor activity upon *in vivo* administration. Other antigens may substitute for CCL21. Immunogenicity can also be assessed by detection of antigen-specific antibodies in a subject's serum, and/or by a demonstration of protective effects of antisera or immune cells specific for the antigen. In some embodiments, a non-pathogenic virus enhances immunogenicity of an antigen by at least about 2-fold, about 5-fold, about 10-fold, about 25-fold, or about 100-fold.

In some embodiments, non-pathogenic viruses are inactivated, as described further herein below. Non-pathogenic viruses, which show anti-cancer, can be subjected to any one of a variety of inactivation methods to render the virus incapable of infecting its native host cell. Using the assays disclosed herein, a skilled artisan can select an inactivation method that preserves anti-cancer activity of the virus. In some embodiments, inactivation methods permit viral entry into host cells, and disrupt transcription and/or replication of the viral genome. In some embodiments, a virus is genetically modified such that it is capable of cellular entry, but is unable to undergo normal transcription and/or replication.

### A2. Anti-infectious Disease Activity of Non-Pathogenic Viruses

Current approaches to treatment of infectious diseases include the use of medicaments that cause adverse or undesirable side effects. Additionally, many effective therapies including vaccination are specific for only a single infectious agent or agents closely related thereto. The inventors of the subject disclosure came to the surprising discovery that non-pathogenic viruses are also potent against infectious diseases. See Example 17.

To select a non-pathogenic virus having anti-infectious disease activity, candidate non-pathogenic viruses can-be tested using an *in vitro* or *in vivo* assay of the infectious disease which are well known by those of skill in the art. For example, for tuberculosis, a rabbit TB model or an *in vitro* Macrophage Model may be used to test for anti-infectious disease activity. Abe et al., (Journal of Immunology, 2003, 171: 1133-1139) discuss other assays suitable for testing compounds for activity against infectious diseases.

In some embodiments, an *in vitro* assay can be used as an initial screen, and then viruses that are active *in vitro* can be subsequently tested in relevant animal models to assess anti-infectious disease activity.

Thus, the present invention provides, *inter alia,* methods for treating a subject in need of anti-infectious disease therapy via administration of a non-pathogenic virus to the subject. Significantly, the non-pathogenic viruses with anti-infectious disease activity disclosed herein do not appear to rely on identification of antigens specific to an infectious agent. Rather, administration of non-pathogenic viruses is broadly applicable.

While the inventors do not wish to be bound to a particular mode of operation, the inventors suggest that the anti-infectious disease activity of non-pathogenic viruses is attributable, at least in part, to their immunostimulatory properties. For example, baculovirus activates dendritic cell maturation and cytolytic T cell (CTL) responses both *in vitro* and *in vivo.* See Examples 9-10.

### B. Therapeutic Applications

The present invention provides methods for treating a cancer-bearing mammalian subject via administration of a non-pathogenic virus to the subject. The disclosed methods are useful for, for example, inhibiting cancer growth, including complete cancer remission, for inhibiting cancer metastasis, and for promoting cancer resistance.

The present invention provides methods for treating a subject having one or more infectious diseases via administration of a non-pathogenic virus to the subject. The disclosed methods are useful for, for example, inhibiting viral replication, inhibit fungal growth.

The term "cancer growth" generally refers to any one of a number of indices that suggest change within the cancer to a more developed form. Thus, indices for measuring an inhibition of cancer growth include but are not limited to a decrease in cancer cell survival, a decrease in tumor volume or morphology (for example, as determined using computed tomographic (CT), sonography, or other imaging method), a delayed tumor growth, a destruction of tumor vasculature, improved performance in delayed hypersensitivity skin test, an increase in the activity of cytolytic T-lymphocytes, and a decrease in levels of tumor-specific antigens.

The term "delayed tumor growth" refers to a decrease in duration of time required for a tumor to grow a specified amount. For example, treatment can delay the time required for a tumor to increase in volume 3-fold relative to an initial day of measurement (day 0) or the time required to grow to 1 cm³.

The term "cancer resistance" refers to an improved capacity of a subject to resist cancer growth, in particular growth of a cancer already had. Alternatively stated, the term "cancer resistance" refers to a decreased propensity for cancer growth in a subject. Cancer resistance is associated with induction of an adaptive immune response, as described herein below.

The term "subject" as used herein includes any mammalian species. In some embodiments, the methods of the present invention are contemplated for the treatment of cancers and/or infectious diseases in mammals such as humans, as well as those mammals of importance due to being endangered, of economical importance and/or social importance to humans.

The term "cancer' generally refers to tumors, including both primary and metastasized tumors. In some embodiments, the tumor is a solid tumor. The term "tumor" encompasses solid tumors and carcinomas of any tissue in a subject, including but not limited to breast; colon; rectum; lung; oropharynx; hypopharynx; esophagus; stomach; pancreas; liver; gallbladder; bile ducts; small intestine; urinary tract including kidney, bladder and urothelium; female genital tract including cervix, uterus, ovaries (e.g., choriocarcinoma and gestational trophoblastic disease); male genital tract including prostate, seminal vesicles, testes and germ cell tumors; endocrine glands including thyroid, adrenal, and pituitary; skin (e.g., hemangiomas and melanomas), bone or soft tissues; blood vessels (e.g., Kaposi's sarcoma); brain, nerves, eyes, and meninges (e.g., astrocytomas, gliomas, glioblastomas, retinoblastomas, neuroma, neuroblastomas, Schwannomas and meningiomas).

The term "tumor" also encompasses solid tumors arising from hematopoietic malignancies such as leukemias, including chloromas, plasmacytomas, plaques and tumors of mycosis fungoides and cutaneous T-cell lymphoma/leukemia, multiple myeloma, and lymphomas including both Hodgkin's and non-Hodgkin's lymphomas.

The term "cancer," as used herein, also encompasses non-neoplastic proliferative disorders. Thus, the methods of the present invention are contemplated for the treatment or prevention of hyperplasia, metaplasia, or most particularly, dysplasia (for review of such abnormal growth conditions, see Robbins & Angell (1976) Basic Pathology, 2d Ed., pp. 68-79, W. B. Saunders Co., Philadelphia, Pennsylvania).

Hyperplasia is a form of controlled cell proliferation involving an increase in cell number in a tissue or organ, without significant alteration in structure or function. As one non-limiting example, endometrial hyperplasia often precedes endometrial cancer. Metaplasia is a form of controlled cell growth in which one type of adult or fully differentiated cell substitutes for another type of adult cell. Metaplasia can occur in epithelial or connective tissue cells. Atypical metaplasia involves a somewhat disorderly metaplastic epithelium. Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exists chronic irritation or inflammation, and is often found in the cervix, respiratory passages, oral cavity, and gall bladder. Although preneaplastic lesions can progress to neoplasia, they can also remain stable for long periods and can even regress, particularly if the inciting agent is removed or if the lesion succumbs to an immunological attack by its host.

Thus, administration of a non-pathogenic virus to a subject as disclosed herein can elicit an innate anti-cancer immune response or an adaptive, cancer-specific immune response. The term "immune system" includes all the cells, tissues, systems, structures and processes, including non-specific and specific categories, that provide a defense against cells comprising antigenic molecules, including but not limited to tumors, pathogens, and self-reactive cells Thus, an immune response can comprise an innate immune response, an adaptive immune response, or a combination thereof.

The term "innate immune system" includes phagocytic cells such as neutrophils, monocytes, tissue macrophages, Kupffer cells, alveolar macrophages, dendritic cells, and microglia. The innate immune system mediates non-specific immune responses. The innate immune system plays an important role in initiating and guiding responses of the adaptive immune system. See e.g., Janeway (1989) Cold Spring Harb Symp Quant Biol 54:1-13; Romagnani (1992) Immunol Today 13:379-381; Fearon & Locksley (1996) Science 272:50-53; and Fearon (1997) Nature 388:323-324. An innate response can comprise, for example, dendritic cell maturation, macrophage activation, cytokine or chemokine secretion, and/or activation of NFKB signaling.

The term "adaptive immune system" refers to the cells and tissues that impart specific immunity within a host. Included among these cells are natural killer (NK) cells and lymphocytes (e.g., B cell lymphocytes and T cell lymphocytes). The term "adaptive immune system" also includes antibody-producing cells and the antibodies produced by the antibody-producing cells.

The term "adaptive immune response" refers to a specific response to an antigen include humoral immune responses (e.g., production of antigen-specific antibodies) and cell-mediated immune responses (.e.g., lymphocyte proliferation), as defined herein below. An adaptive immune response can further comprise systemic immunity and humoral immunity.

The terms "cell-mediated immunity" and "cell-mediated immune response" refer to the immunological defense provided by lymphocytes, such as that defense provided by T cell lymphocytes when they come into close proximity to their victim cells. A cell-mediated immune response also comprises lymphocyte proliferation. When "lymphocyte proliferation" is measured, the ability of lymphocytes to proliferate in response to specific antigen is measured. Lymphocyte proliferation is meant to refer to B cell, T-helper cell or CTL cell proliferation.

The term "CTL response", as used herein refers to the ability of an antigen-specific cell to lyse and kill a cell expressing the specific antigen. As described herein, standard, art-recognized CTL assays are performed to measure CTL activity.

The term "systemic immune response", as used herein, refers to an immune response in the lymph node-, spleen-, or gut-associated lymphoid tissues wherein cells, such as B lymphocytes, of the immune system are developed. For example, a systemic immune response can comprise the production of serum immunoglobulins (IgGs). Further, systemic immune response refers to antigen-specific antibodies circulating in the blood stream and antigen-specific cells in lymphoid tissue in systemic compartments such as the spleen and lymph nodes.

The terms "humoral immunity" or "humoral immune response" are meant to refer to the form of acquired immunity in which antibody molecules are secreted in response to antigenic stimulation.

The term "cancer-specific," as used herein to describe an adaptive immune response, refers to a cell-mediated or humoral immune response in a subject, wherein the response is directed specifically to a cancer previously present in the subject. Given that innate and adaptive immune responses involve unique immune cell types, one would not expect that methods for eliciting an innate immune response could also elicit an adaptive immune response. In some embodiments, administration of a non-pathogenic virus to a subject elicits both an innate immune response and an adaptive immune response.

In some embodiments, the methods disclosed herein for the administration of non-pathogenic viruses can be combined with one or more other cancer therapies. For example, a tumor or abnormal cell growth can be surgically removed before or after administration of a non-pathogenic virus. Similarly, a non-pathogenic virus of the invention can be co-administered or co-formulated with additional agents, for example anti-angiogenic, chemotherapeutics, and/or additional immunomodulatory agents. Representative agents that can be used in conjunction with a non-pathogenic virus include, but are not limited to, methoxtrexate, tamoxifen, nelandron, nilutamide, adriamycin, 5-fluorouracil (5FU), cytokines such interferon alpha (IFN-α), interferon gamma (IFN-γ), interleukin 2 (1L2), interleukin 4 (IL4), interleukin 6 (IL6), and tumor necrosis factor (TNF). Infectious diseases can further be treated by administering anti-virals, anti-biotics, or anti-fungals.

The present invention further relates to methods and compositions useful for inducing cytotoxic T-cell mediated responses in mammalian subjects, including humans. In some embodiments, the present invention relates to the use of a non-pathogenic virus for inducing cytotoxic T-cell mediated responses. Thus, the present invention provides methods for preparing antigen formulations comprising a non-pathogenic virus and an antigen. The term "antigen" refers to a substance that activates lymphocytes (positively or negatively) by interacting with T cell or B cell receptors. Positive activation leads to immune responsiveness, and negative activation leads to immune tolerance. An antigen can comprise a protein, a carbohydrate, a lipid, a nucleic acid, or combinations thereof. An antigen can comprise a heterologous (e.g., an antigen that is typically not found in a host subject) or an autologous antigen (self antigen).

Also provided are methods for using the disclosed antigen formulations as therapeutic and/or prophylactic agents. For example, such antigen formulations can be administered to a mammalian subject for the treatment of diseases in which a CTL response is important, for example, in the treatment of HIV infection or influenza; it can also be extended to use in treatment or prevention of bacterial infections, parasitic infections, and the like.

In some embodiments the present invention provides methods of inhibiting one or more symptoms of cancer in an individual in need thereof. The methods comprise administering to the individual an amount of a composition comprising a non-pathogenic virus effective to inhibit one or more symptoms of cancer in the individual.

Symptoms of cancer are well known to the art-skilled and include both physiological and physical indicia. Physiological indicia includes, without limitation, tumor growth, abnormal cell growth, metastasis, angiogenesis, cell death or cell invasiveness. Physical indicia include, without limitation, weight loss, bleeding, difficulty in breathing, bone fractures, compromised immune system or fatigue.

The administration of a non-pathogenic virus to a subject as disclosed herein can also elicit an anti-infectious disease immune response. As discussed above, an immune response can comprise an innate immune response, an adaptive immune response, or a combination thereof.

The present invention also provides methods of protecting an animal from an infectious disease comprising administering to the animal an effective amount of a composition comprising a non-pathogenic virus. In some embodiments the non-pathogenic virus is inactivated by any methods or by methods disclosed herein. Based on the Examples described below it was observed that the administration of inactive non-pathogenic virus protected against an infectious agent (e.g. virus, fungus, or bacteria). In some embodiments, the non-pathogenic virus is an insect-specific virus (e.g. Baculaviridae family). The non-pathogenic virus can also be co-administered with other vaccines, anti-viral, anti-fungal, ant-bacterial, or combinations thereof.

### C. Therapeutic Compositions and Methods

The present invention further provides pharmaceutical compositions and methods for using the same. A non-pathogenic virus of the invention is prepared and formulated for safe and efficacious anti-tumor and/or anti-infectious disease and/or adjuvant activity, as described herein.

The present invention also provides compositions that can be used to treat or prevent infectious disease and/or cancer. In some embodiments, the compositions comprise a non-pathogenic virus and peripheral blood mononuclear cells (PBMCs). In some embodiments, the PBMCs are isolated from the animal or individual, contacted with the non-pathogenic virus ex *vivo,* and then re-administered to the animal or individual as a mixture or combination. In some embodiments, the PBMCs are isolated from a different animal or individual than is being treated or to whom the compositions of the present invention are being administered.

In some embodiments, the composition comprises non-pathogenic virus and at least one tumor cell. The tumor cell can either be autologous or allogenic to the individual or animal.

In some embodiments, the composition comprises a non-pathogenic virus, at least one tumor cell, and at least one PBMC. In some embodiments the non-pathogenic virus is an inactive virus.

### C.1. Viral Inactivation

In some embodiments, live non-pathogenic viruses used in the methods of the present invention are inactivated prior to administration to a subject Non-pathogenic viruses, as defined herein above, are incapable of replication in a mammalian host. Inactivation, which renders the virus non-replicative in its native host cell, can be performed as an additional safety measure.

Viral inactivation can be accomplished by any suitable means, including but not limited to destruction of lipid or protein components of a viral coat, modification such that the virus is unrecognizable to a target cell, destruction of viral nucleic acid, and/or rendering of the virus as irreplicable. Representative methods for viral inactivation include but not limited to pasteurization, treatment with detergents (e.g., Triton-X100®), alkylation with binary ethylenimine (BEI), photochemical inactivation, UV-light inactivation, radiation, physical disruption (e.g. sonication, electron beam); genetic inactivation and combinations thereof. See Rueda et al. (2000) Vaccine 19:726-34 and Henzler & Kaiser (1998) Nat Biotechnol 16:1077-9. in some embodiments, inactivation does not significantly reduce viral antigenicity and/or activity. Viral inactivation is assayed using standard methods for determining viral infectivity.

"Genetic inactivation," as used herein, refers to the manipulation of the nucleic acids (e.g. genes) of the virus. The manipulation can include, for example, deletion of one or more genes, mutation of at least one gene; creation of temperature sensitive mutants, inactivation of a gene, and the like. Temperature sensitive mutants are mutants that at one temperature are permissive, while at another temperature it is restrictive (e.g. inhibits viral replication). In the case of a baculovirus this can be used to allow the virus to grow at room temperature (e.g. about 25°C) for propagation and preparation, but when administered to an animal the higher internal temperature of the animal will inactivate the virus. In some embodiments, the temperature sensitive mutant will be permissive at a temperature in the ranges of about 16-28°C, about 20-28°C, about 25-28°C, or about 27°C. In some embodiments, the restrictive temperature for a temperature sensitive mutant is about 30-45°C, about 32-40°C, about 35-38°C, about 37°C. In some embodiments the restrictive temperature is any temperature above about 28°C, about 29°C, about 30°C, about 31°C, about 32°C, about 33°C, about 34°C, about 35°C, about 36°C, or about 37°C. In some embodiments the temperature sensitive mutant is inactive inside an animal or an individual. In some embodiments, the temperature sensitive mutant is 100% less active, about 90% less active, about 80% less active, about 70% less active, about 60% less active, about 50% less active, about 40% less active, about 30% less active, about 20% less active, or about 10% less active as compared to the wild-type virus at the restrictive temperature.

Temperature sensitive mutants can have any gene mutated that reduces the activity of the virus at one temperature when compared to another temperature. Examples of genes or proteins that can be mutated include, but are not limited to Guanylyltransferase, RNA triphosphatase, ATPase, a protein kinase (e.g. PK-1), and the like. Examples of temperature sensitive mutants can be found in, for example, Jin et al., Journal of Virology, (1998), Vol. 72, pp.10011-10019, and McLachlin et al., Virology, (1998). Vol. 246, pp. 379-391, each of which are hereby incorporated by reference.

Pasteurization is a simple approach for inactivation if the viruses can withstand thermal treatment sufficient for inactivation. In some embodiments, the heating is performed for a minimally sufficient time period to minimize damage to viral proteins. Optionally, viral damage can be minimized by the use of stabilizers and sodium citrate, saccharose, and/or glycine.

Alternately, chemical inactivation, for example mild pepsin processing at low pH values or exposure to detergents, can be used to disrupt the lipid bilayer and thus can be used for inactivating enveloped viruses, including baculovirus. See U.S. Patent Nos. 4,820,805 and 4,764,369. Aziridine binary ethylenimine is a potent alkylating agent that inactivates virus by selectively interacting with nucleophylic groups of nucleic acids but not proteins.

In some embodiments of the invention, viral inactivation is achieved via a photochemical reaction. According to this approach, a radiation sensitizing chemical compound is added to a liquid suspension of non-pathogenic viruses, and the mixture is exposed to UV light or ionizing (y or X-ray) radiation.

Psoralen, and derivatives thereof, and compounds with a linear tricyclic structure resembling psoraien, are capable of evoking photosensitization. Psoralens are bi-functional photoreactive molecules, which form covalent bonds with nucleic acids in the presence of long wavelength ultraviolet light. Psoralen molecules intercalate into DNA duplexes and then photoreact to cross-link the individual strands of the DNA. See Hwang et al. (1996) Biochem Biophys Res Commun 219:191-7. The crosslinking renders the DNA unable to replicate or to be transcribed. Commercially available psoralen compounds include 8-methoxypsoralen (methoxsalen) and 4,5', 8 trimethyl psoralen (trioxalen). The wavelengths most effective for photochemical inactivation using psoralen are in the range between 320 nm and 380 nm, with maximum effectiveness between 33 nm and 360 nm. See Pathak, M (1974) in Sunlight and Man, eds. Pathak, M & Fitzpatrick, T, University of Tokyo Press, Tokyo.

Additional photosensitizing agents include halogenated psoralens, angelicins, khellins and coumarins, which each contain a halogen substituent and a water solubilization moiety, such as, quaternary ammonium ion or phosphonium ion. It is believed that the substitution of halogen atoms, particularly bromine atoms, on psoralen molecules increases the binding constant of the sensitizer to DNA due to the hydrophobic nature of bromine. In some embodiments, brominated photosensitizing agents are used because only one photon of light is required to activate the brominated sensitizer, whereas two photons are required to effect DNA crosslinking using non-brominated psoralens. See, for example, U.S. Patent No. 5,418,130.

A representative method for photochemical inactivation is described in Example 11, which employs a combination of trioxalen and long wavelength UV illumination. See, for example, Weightman & Banks (1999) J Virol Methods 81:179-82 and Cotten at al. (1992) Proc Natl Acad Sci USA 89:6094-8.

To preserve antigenic characteristics of the virus, psoralen inactivation of live virus can be performed in a non-oxidizing atmosphere. By excluding oxygen and other oxidizing species from the inactivation medium, degradation of antigens via irradiation with ultraviolet light is largely prevented. See U.S. Patent No. 5,106,619. Similarly, antioxidants/quenchers can be used to minimize free radicals and other reactive oxygen species that are generated by exposure to short wave length UV light, and to thereby minimize protein damage. See, for example, Marx et al. (1996) Photochem Photobiol 63:541-6.

In some embodiments of the invention, viral inactivation comprises modification of viral genes, whereby the virus is impaired or unable to replication. For example, a virus can be genetically modified to include one or more temperature-sensitive mutations in viral essential genes. The virus is produced and grown in Sf9 or Tn5 cultures at the permissive temperature (e.g., 25°C). When the virus is introduced into a mammal subject during treatment, the temperature is non-permissive (e.g., 37°C) such that the temperature-sensitive genes would be poorly expressed, or the resultant gene products would have impaired function, and the virus would be crippled.

Representative temperature-sensitive mutations that could be generated include genes that are required for viral infection. For example, temperature-sensitive mutations in the gene encoding PKIP, a protein which interacts with a virus-encoded protein kinase, and in regulators of viral late gene transcription. At the non-permissive temperature, virus bearing such mutations show defects in viral infection. See, for example, McLachlin et al. (1998) Virology 246:379 and Partington et al. (1990) Virology 175:91.

Virus inactivation can be assessed by demonstrating a loss in ability to replicate in a native host cell. Infectivity of a sample can be demonstrated using a standard plaque assay. When suitable methods to demonstrate infectivity of a particular virus are unknown, assessment of inactivation can rely on demonstrating inactivation of a model virus having similar biophysical and structural qualities. See Henzler & Kaiser (1998) Nat Biotechnol 16:1077-9. To render a virus completely inactive, the inactivation methods used in accordance with the present invention can include sequential exposure to an inactivating stimulus.

### C.2. Carriers

As described herein, a non-pathogenic virus can comprise a live virus, an inactivated virus, a viral particle, a viral occlusion body, a viral component, or combinations thereof. To facilitate delivery of a non-pathogenic virus to cancer cells in a subject, a composition that is administered to elicit an anti-cancer and/or an anti-infectious disease response in a subject comprises: (a) an effective amount of a non-pathogenic virus; and (b) a pharmaceutically acceptable carrier. Where appropriate, two or more carriers can be used together.

As used herein, the term "carrier" refers to a compound or a group of compounds that can be used to transport a virus, virus like particle, viral component, viral protein to or across a plasma membrane of a cell.

Representative carriers for delivery of a non-pathogenic virus or viral component include, for example, liposomes, nanospheres (Manome et al., 1994; Saltzman and Fung, 1997), a glycosaminoglycan (e.g., U.S. Patent No. 6,106,866), fatty acids (e.g., U.S, Patent No. 5,994,392), fatty emulsions (e.g., U.S. Patent No. 5,651,991), lipids and lipid derivatives (e.g., U.S. Patent No. 5,786,387), collage (e.g., U.S. Patent No. 5,922,356), polysaccharides and derivatives thereof (e.g., U.S. Patent No. 5,688,931), nanosuspensions (e.g., U.S. Patent No. 5,858,410), polymeric micelles or conjugates (e.g., U.S. Patent Nos. 4,551,482, 5,714,166, 5,510,103, 5,490,840, and 5,855,900), and polysomes (e.g., U.S. Patent No. 5,922,545),

For delivery of a viral component, the carrier can further comprise a gene therapy vector, including, for example, a viral vector or a plasmid vector. Suitable viral vectors for gene expression include adenoviruses, adeno-associated viruses (AAVs), retroviruses, pseudotyped retroviruses, herpes viruses, vaccinia viruses, and Semiliki forest virus. A carrier can also include, for example, a virus like particle, protein delivery vehicles including, for example, Pro-Ject (pierce Biotechnology, Inc.) and Profect (Targeting Systems), and Chariot™ (Active Motif), and the like.

A carrier can be selected to effect sustained bioavailability of a non-pathogenic virus to a site in need of treatment. The term "sustained bioavailability" encompasses factors including but not limited to prolonged release of a non-pathogenic virus from a carrier, metabolic stability of a non-pathogenic virus, systemic transport of a composition comprising a non-pathogenic virus, and effective dose of a non-pathogenic virus.

Representative compositions for sustained bioavailability can include but are not limited to polymer matrices, including swelling and biodegradable polymer matrices, (U.S. Patent Nos. 6,335,035; 6,312,713; 6,296,842; 6,287,587; 6,267,981; 6,262,127; and 6,221,958), polymer-coated microparticles (U.S. Patent Nos. 6,120,787 and 6,090,925) a polyol;oil suspension (U.S. Patent No. 6,245,740), porous particles (U,S. Patent No. 6,238,705), latex/wax coated granules (U.S. Patent No. 6,238,704), chitosan microcapsules, and microsphere emulsions (U.S. Patent No. 6,190,700).

### C.3. Formulation, Dose and Administration

Suitable formulations for administration of a composition of the invention to a subject include aqueous and non-aqueous sterile injection solutions which can contain anti-oxidants, buffers, bacteriostats, antibacterial and antifungal agents (e.g., parabens, chlorobutanol, phenol, ascorbic acid, an thimerosal), solutes that render the formulation isotonic with the bodily fluids of the intended recipient (e.g., sugars, salts, and polyalcohols), suspending agents and thickening agents. The formulations can be presented in unit-dose or mulfi-dose containers, for example sealed ampoules and vials, and can be stored in a frozen or freeze-dried (lyophilized) condition requiring only the addition of sterile liquid carrier immediately prior to use.

Compositions useful for injection into a host include sterile aqueous solutions or dispersions, and sterile powder for the preparation of sterile injectable solutions or dispersions. An injectable composition should be fluid to the extent that administration via a syringe is readily performed. Suitable solvents include water, ethanol, polyol (e.g., glycerol, propylene glycol, and liquid polyethylene glycol), and mixtures thereof. Fluidity can be maintained, for example, by the use of a coating such as lecithin and/or by minimization of particle size.

A non-pathogenic virus of the present invention can be administered to a subject intratumorally, peritumarally, systemically, parenterally (e.g., intravenous injection, intra-muscular injection, intra-arteeial injection, and infusion techniques), orally, transdermally (topically), intranasally (inhalation), and intramucosally. A delivery method is selected based on considerations such as the type of the type of carrier or vector, therapeutic efficacy of the composition, location of target area, and the condition to be treated.

As used herein, the term "protein delivery vehicle" refers to an agent(s) that facilitates the transport of a protein to or across the membrane of a cell.

In some embodiments, a non-pathogenic virus is administered by direct injection into a tumor or into a peritumor site. The term "peritumor site" refers to a site less than about 15 cm from an outer edge of a tumor, less than about 10 cm from an outer edge of a tumor, less than about 5 cm from an outer edge of a tumor, less than about 1 cm from an outer edge of a tumor, or less than about 0.1 cm from an outer edge of a tumor. A non-pathogenic virus of the invention can be delivered to one or more tumor and/or peritumor sites. In some embodiments, a non-pathogenic virus of the invention is administered at multiple sites within a tumor and/or surrounding a tumor.

In some embodiments, wherein the cancer is a non-neoplastic growth a non-pathogenic virus is administered at a lesional or perilesional site. The term "perilesional site" refers to a site less than about 15 cm from an outer edge of a non-neoplastic growth, less than about 10 cm from an outer edge of a non-neoplastic growth, less than about 5 cm from an outer edge of a non-neoplastic growth, less than about 1 cm from an outer edge of a non-neoplastic growth, or less than about 0.1 cm from an outer edge of a non-neoplastic growth. A non-pathogenic virus of the invention can be delivered to one or more lesional and/or perilesional sites, In some embodiments, a non-pathogenic virus of the invention is administered at multiple sites within a non-neoplastic growth and/or surrounding a non-neoplastic growth.

In some embodiments, wherein the compositions are being used to treat an infectious disease, a non-pathogenic virus is administered systemically. In some embodiments a non-pathogenic virus is administered locally to affected regions.

The present invention provides that an effective amount of a non-pathogenic virus is administered to a subject. The term "effective amount" is used herein to describe an amount of a non-pathogenic virus sufficient to elicit anti-cancer activity, including, for example, an anti-tumor activity and/or an anti-non-neoplastic growth activity. As disclosed herein, representative anti-cancer activities include but are not limited to cancer cell cytolysis, inhibition of cancer growth, inhibition of cancer metastasis, and/or cancer resistance, In some embodiments, an "effective amount" refers to the amount of a therapeutic that is effective in an *in vitro* assay in inhibiting cancer growth, inhibiting metastasis, inhibiting cancer resistance, inducing cell cytolysis, inducing cell death, and the like. In some embodiments, an "effective amount" inhibits cancer growth, inhibits metastasis, inhibits cancer resistance, induces cell cytolysis, induces cell death, or combinations thereof at least 10%, at least 20%, at least 30%, at least 40%, at least 50%, at least 60%, at least 70%, at least 80%, at least 90%, at least 2-fold, at least 5-fold, at least 10-fold, or at least 100-fold.

The term "effective amount" is used herein to describe an amount of a non-pathogenic virus sufficient to elicit anti-infectious disease activity. In some embodiments, an "effective amount" inhibits viral replication at least 10%, at least 20%, at least 30%, at least 40%, at least 50%, at least 60%, at least 70%, at least 80%, at least 90%, at least 2-fold, at least 5-fold, at least 10-fold, or at least 100-fold, compared to a control.

Actual dosage levels of active ingredients in a therapeutic composition of the invention can be varied so as to administer an amount of the composition that is effective to achieve the desired therapeutic response for a particular subject. Administration regimens can also be varied as required to elicit the desired activity. A single injection or multiple injections can be used. The selected dosage level and regimen will depend upon a variety of factors including the activity of the therapeutic composition, formulation, the route of administration, combination with other drugs or treatments, the disease or disorder to be treated, and the physical condition and prior medical history of the subject being treated. Determination and adjustment of an effective amount or dose, as well as evaluation of when and how to make such adjustments, are known to those of ordinary skill in the art of medicine.

The dose of a non-pathogenic virus can be calculated by a variety of methods. For a live non-pathogenic virus the dose can be calculated as plaque-forming units. For an inactive non-pathogenic virus, which does not form plaques, the amount of virus administered to an individual can be measured in terms of PFU equivalents. As used herein, the term "PFU equivalent" refers to a quantity of non-pathogenic virus. A PFU equivalent is defined as the amount of virus resulting after 1 PFU of a virus is inactivated.

Another method of determining quantity of virus to be administered is based on the number of viral particles present in a sample. The particles can be counted by any method including, for example, electron microscopy. The non-pathogenic virus, including inactivated virus, can also be administered using an amount extrapolated from an amount effective in an *in vitro* assay. The in *vitro* assay can be any assay known to those skilled in the art for measuring anti-cancer or anti-infectious disease activity, including, but not limited to, assays that measures cytotoxicity, cell death, ability of cells to grow in soft-agar, and the like. In some embodiments, the dose is the amount that increases cytotoxicity or cell death by at least 20%, at least 30%, at least 40%, at least 50%, at least 60%, at least 70%, at least 80%, at least 90%, or at least 99%, relative to a control. In some embodiments, the does is the amount of virus that decreases the ability of cells to grow in soft-agar by at least 20%, at least 30%, at least 40%, at least 50%, at least 60%, at least 70%, at least 80%, at least 90%, or at least 99%, relative to a control.

For additional guidance regarding formulation, dose and administration regimen, see Berkow et al. (1997) The Merck Manual of Medical Information, Home ed. Merck Research Laboratories, Whitehouse Station, New Jersey; Goodman et al. (1996) Goodman & Gilman's the Pharmacological Basis of Therapeutics, 9th ed, McGraw-Hill Health Professions Division, New York; Ebadi (1998) CRC Desk Reference of Clinical Pharmacology. CRC Press, Boca Raton, Florida; Katzung (2001) Basic & Clinical Phamacology, 8th ed. Lange Medical Books/McGraw-Hill Medical Pub. Division, New York; Remington et al. (1975) Remington's Pharmaceutical Sciences, 15th ed. Mack Pub. Co., Easton, Pennsylvania; Speight et al. (1997) Avery's Drug Treatment; A Guide to the Properties, Choice, Therapeutic Use and Economic Value of Drugs in Disease Management, 4th ed. Adis International, Auckland / Philadelphia, Pennsylvania.

In some embodiments, compositions are tested *in vitro* or *in vivo* assays in order to determine an "effective amount" For example, in methods disclosed herein for causing cell death, assays suitable include, Without limitation, *in vitro* cell viability assays, including the TUNEL assay or other fluorescent based assays such as Cell-Titer Blue (Promega Corp); assays that monitor DNA fragmentation; and cytochrome C release assays, soft-agar growth assays, contact inhibition assays, and tumor growth in nude mice; assays comprising injecting a test animal with a tumor monitoring cancer remission following administration of the compositions of the present invention and transgenic mice assays wherein the transgenic mice have tumors and cancer remission is monitored; the *in vivo* assay disclosed herein; *in vitro* assays measuring cell movement across a barrier, such as Matrigel barrier (see, for example, Cancer Res. 2003 Aug 1;63(15):4632-40; Am J Chin Med. 2003;31(2):235-46); in *vitro* Invasiveness and in *vivo* Metastasis Assays discussed in Yang et al., (Cancer Res. 61, 5284-5288, July 1, 2001).

The compositions of the present invention comprising a non-pathogenic virus may further comprise one or more adjuvants which may be used to enhance the effectiveness of the pharmaceutical compositions. Such adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, Mass.), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi^{™}. adjuvant system (RAS), (Ribi Immunochem, Hamilton, Mont.) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL+CWS (Detox^{™}) (for a further discussion of suitable submicron oil-in-water emulsions for use herein, see International Publication No. WO 99/30739, published Jun. 24, 1999); (3) saponin adjuvants, such as Stimulon^{™} (Cambridge Bioscience, Worcester, Mass.) may be used or particle generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), etc.; (6) detoxified mutants of a bacterial ADP-ribosylating toxin such as a cholera toxin (CT), a pertussis toxin (PT), or an E. coli heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), adjuvants derived from the CpG family of molecules, CpG dinucleotides and synthetic oligonucleotides which comprise CpG motifs (see, e.g., Krieg et al., Nature, 374:546 (1995) and Davis et al., J. lmmunol., 160:870-876 (1998)) and PT-K9/G129 (where lysine is substituted for the wild-type amino acid at position 9 and glycine substituted at position 129) (see, e.g., International Publication Nos. WO93/13202 and WO92/19265); and (7) other substances that act as immunostimulating agents to enhance the effectiveness of the composition.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2'-dipahitoyl-sn-glycero-3-huydroxyphosphoryloxy)-ethylamine (MTP-PE), etc.

International Publication No. WO 98/50071 describes the use of viral-like particles (VLPs) as adjuvants to enhance immune responses of antigens administered with the VLPs. St. Clair et al. describe the use of protein crystals to enhance humoral and cellular responses. (St. Clair, N. et al, Applied Biol. Sci., 96:9469-9474, 1999).

It will be readily apparent to one skilled in the art that following a review of the present disclosure, the therapeutic methods for administration of a non-pathogenic virus can be variably performed to elicit an anti-cancer response and/or an anti-infectious disease response.

In accordance with long-standing convention, the terms "a," "an," and "the" are used to refer to one or more. The term "about", as used herein when referring to a measurable value, for example a peritumoral or perilesional distance, is meant to encompass variations of ±20% or ±10%, ±5%, ±1%, or ±0.1% from the specified amount, as such variations are appropriate to perform a disclosed method or otherwise carry out the present invention. In some embodiments "about" is meant to encompass variations of ±10% from the specified amount.

### D1. Predicting In vivo Activity

Predicting *in vivo* anti-tumor activity is often difficult and haphazard. The present invention provides methods for predicting *in vivo* anti-tumor activity. In some embodiments, the method comprises contacting a compound with tumor cells and peripheral blood mononuclear cells and measuring cell death of the tumor cells. In some embodiments, the compound is a non-pathogenic virus or a non-pathogenic insect-specific virus. Any method can be used to measure cell death including, for example, measuring apoptosis, necrosis, cell viability, and the like. Examples of tumor cells that can be used, include but are not limited to, are lung cancer cells (e.g. A549 cells, 3LL-HM cells), breast cancer cells (e.g. 4T1 cells; MT901 cells; MAT Bill cells), prostate cancer cells, colon cancer cells, skin cancer cells (e.g. B16 melanoma cells), pancreas cancer cells, liver cancer cells, brain cancer cells, bone cancer cells (e.g. MG-63 cells), stomach cancer cells, or esophageal cancer cells, among others.

**E.1 Release Assays**

Prior to release to the public of medicaments, governmental agencies, for example, the Food and Drug Administration (FDA), frequently require that stringent quality control be performed on such medicaments to ensure that the medicaments comprise the same ingredients as were contained in medicaments approved by that or other governmental agencies. Often, such quality control is performed using release assays-assays to ensure the medicaments meet regulatory guidelines.

The present invention provides processes for preparing anti-cancer and anti-infectious disease compositions for release. In some embodiments the compositions comprise one or more non-pathogenic viruses. In some embodiments the non-pathogenic virus is an *Autographa californica* nucleopolyhedrosis virus. In some embodiments the non-pathogenic virus comprises an inactivated virus, a viral particle, a virosome, a Virus Like Particle, a viral occlusion body, or a viral component. In some embodiments the processes comprise performing release assays on the compositions to ensure that the safety, toxicity and efficacy of the compositions meet proscribed guidelines.

The processes for preparing anti-cancer and anti-infectious disease compositions comprise exposing the composition to a first inactivator effective to inactivate an active virus, exposing the composition to a second inactivator effective to inactivate an active virus, combining the composition with one or more pharmaceutically acceptable carriers or excipients, and confirming the inactivity of the inactivated virus, viral particle, virosome, Virus Like Particles, viral occlusion body, or viral component in an *in vitro* assay. In some embodiments the confirmation of the inactivity of said virus, viral particle, virosome, Virus Like Particles, viral occlusion body, or viral component is performed after each of the inactivating steps.

In some embodiments the processes further comprise collecting a random portion of the composition for analysis of one or more of safety, efficacy, or toxicity. In some embodiments the safety and/or efficacy and/or efficacy of the random portion is compared to the safety and/or efficacy and/or efficacy of a second anti-cancer or anti-infectious disease composition. The comparison data is generated for review prior to release.

In some embodiments the inactivity of the inactivated virus, viral particle, virosome, Virus Like Particles, viral occlusion body, or viral component is confirmed by plaque formation assay. In some embodiments the plaque formation assay is performed with Sf9 cells, In some embodiments the process further comprises counting the inactivated virus, viral particle, virosome, Virus Like Particles, viral occlusion body, or viral component. Counting may be performed by any method known to those of skill in the art. In some embodiments Counting is performed using EM.

The following examples are meant to illustrate the invention and are not to be construed to limit the invention in any way. Those skilled in the art will recognize modifications that are within the spirit and scope of the invention,

### Examples

**Example 1. Preparation of Recombinant Baculovirus**

The full-length sequence encoding human CCL21 (GenBank Accession No. NM 002989) was cloned into the baculovirus transfer vector pVL1392 (Pharmingen of San Diego, California) and ca-transfected with BACULOGOLD® WT genomic DNA (Pharmingen of San Diego, Califomia) using methods recommended by the vendor. The recombinant baculovirus obtained from this procedure was subcloned by plaque-purification on Sf9 insect cells to yield several isolates expressing human CCL21. A clone was selected for its exceptional expression characteristics compared to the original virus. Amplification of this baculovirus isolate was performed at low multiplicity of infection (MOI) to generate high-titer, low passage stock for protein production. The baculovirus expressing human CCL21 was designated BV422. Additional recombinant baculovirus were similarly prepared. For example, baculovirus expressing intracellular Raf protein was prepared and designated BV762.

Protein production and budded baculovirus production involved infection of suspended Trichoplusia ni (Tn5) cells in protein-free media at multiplicity of infection (MOI) of 2-10 for 48 hours. BV422 culture supernatants, which included the recombinantly expressed CCL21, were collected by centrifugation, clarified by filtration and prepared for column purification.

**Example 2. Tumor Growth Inhibition in an Animal Model of Lung Cancer**

C57BL/6 mice at 9-11 weeks of age were allowed to acclimate for a minimum of 7 days prior to inoculation with tumor cells. Mice were inoculated s.c. at the right flank with 2 X 10⁵ early passage (<10 passages) 3LL-HM tumor cells. Tumor size was measured twice per week. When tumors reached 50-100 mm³ (typically 7 days after tumor inoculation), mice were randomized into groups. Baculovirus-expressed CCL21 was administered intratumorally to tumor-bearing mice. Dose and administration regimens were varied to optimize tumor inhibition. When tumor volume in any group reached 3000 mm³ (typically 33-35 days after inoculation in mice of the control group), mice were sacrificed.

As shown in Figure 1, intratumoral administration of baculovirus-expressed CCL21 resulted in growth delay of 3LL tumors. CCL21 dose was optimized to achieve complete inhibition of tumor growth. An administration regimen that included 2 or 3 injections at a relatively higher dose showed similar efficacy when compared to an administration regimen that included 6 injections at a relatively lower dose. Some tumor inhibition was also seen using a single dose.

**Example 3. Tumor Growth Inhibition in an Animal Model of Breast Cancer**

Balb/c mice at 9-11 weeks of age were allowed to acclimate for a minimum of 7 days prior to inoculation with tumor cells. Mice were inoculated s.c. at the right flank with 2 X 105 4T1 cells. Tumor size was measured twice per week. When tumors reached 50-100 mm³ (typically 7 days after tumor inoculation), mice were randomized into groups. Baculovirus was administered intratumorally to tumor-bearing mice. Dose was varied to determine an optimal effective dose. When tumor volume in any group reached 3000 mm³ (typically 33-35 days after inoculation in mice of the control group), the mice were sacrificed. As shown in Figure 2, intratumoral administration of CCL21 resulted in growth delay of 4T1 tumors,

**Example 4. Tumor Growth Inhibition in a Melanoma Model**

The mouse melanoma cell line, B15-BL6, is used to establish subcutaneous tumors in 6-8 week old pink-skinned female BDF-1 mice (Charles River Laboratories of Boston, Massachusetts). To produce cutaneous tumors, 106 B16-BL6 cells in 0.2 ml media are injected into the upper back region of 6-8 week old female BDF-1 mice on day 0. Cell viability is assessed using trypan blue exclusion before and after cell injection. The number of dead cells before injection is typically not more than 10% of the total number of cells. By day 6, tumors are typically 5-10 mm in diameter.

Baculovirus-expressed CCL21 are prepared as described in Example 1. Baculovirus are administered subcutaneously, at a site approximately 3 mm away from each tumor, on days 3 and 4. Tumor volume is measured daily for three weeks. Mice are sacrificed when tumor volume reaches 4000 mm³.

**Example 5. Resistance to Tumor Re-challenge**

Mice bearing tumors were prepared and treated with Baculovirus as described above. Mice having complete tumor regression or in mice that did not have complete tumor regression had their tumors surgically removed 2 days after the final dose of Baculovirus were subjected to tumor rechallenge. Mice were anesthetized using 200 µl ketamine/xylazine mixture (4:1 ketamine:xylazine diluted 10-fold in phosphate-buffered saline) injected intraperitoneally, the tumor was resected, and the wound was closed with staples. One to four days following tumor resection, mice were rechallenged by subcutaneous administration of 2 X 10⁵ 4T1 cells at a site other than the original tumor site. Rechallenge tumor volume was measured twice per week. In mice having complete tumor regression, the mice were regrouped evenly into two groups. One group was rechallenged with the same tumor cells at the opposite side of flank at 1X10⁵ cells per mouse. The other group was challenged with different syngeneic tumor cells (B16F10) at the left flank at 1X10⁵ cells per mouse. Tumor development on the re-challenged site was monitored. Mice treated with Baculovirus were able to resist rechallenge with 3LL-. HM tumor cells, but were not resistant to the different syngeneic tumor cells.

**Example 6. Inhibition of Tumor Metastases**

Mice bearing tumors were prepared and treated with baculovirus as described in Example 3. Mice were sacrificed when the control group showed signs of severe sickness due to lung metastases. Typical indicators include laborious breathing, greasy fur, and weight loss. The lungs were harvested and preserved in Buoin's solution. The presence of lung metastases was determined. Figure 3 shows that baculovirus-expressed CCL21 significantly inhibited tumor metastasis.

**Example 7. Anti-Tumor Activity of Baculovirus**

The anti-tumor activity of baculovirus-expressed hCCL21, as described in Examples 2-3 and 5-6, is attributable to baculovirus rather than to CCL21. As shown in Figure 6, filtered preparations of baculovirus-expressed CCL21 were insufficient to effect tumor remission in a 3LL tumor model. The concentration of CCL21 in any given sample was unchanged by filtration. Some but not all preparations of baculovirus-expressed CCL21 were similarly ineffective.

In contrast to the variability observed *in vivo,* all CCL21 preparations were sufficient to induce chemotaxis of lymphocytes *in vitro.* See Table 1 and Figure 7. These results suggest that a high molecular weight contaminating substance in baculovirus-expressed CCL21 was required for robust anti-tumor activity, but not for CCL21-induced chemotaxis *in vitro.*

**Table 1. Activity of Recombinant CCL21 Preparations**

| **CCL21** **Species of Origin** | **Host for** **Heterologous Expression** | **Lot#** | ***In vitro*** **chemotaxis activity** | ***In vivo*** **anti-tumor activity** |
|---|---|---|---|---|
| Mouse | Baculovirus | MBAY1 | + | + |
| Mouse | Baculovirus | MBMC1 | + | + |
| Mouse | Baculovirus | MBDS1 | + | - |
| Mouse | Baculovirus | MBDS2 | + | + |
| Human | Baculovirus | HBDS1 | + | - |
| Human | Baculovirus | HBMC1 | + | - |
| Human | Baculovirus | HBDS2 | + | + |
| Human | Baculovirus | HBDS3 | + | + |
| Human | Baculovirus | HBPG1 | + | + |
| Human | Baculovirus | HBDS4 | + | + |
| Mouse | *E. coli* | PeproTech | + | - |
| Human | *E. coli* | HEDS1 | + | - |
| Human | *E. coli* | HERDS4 | + | - |
| Human | *E. coli* | HEPG3 | + | - |
| Human | *E. coli* | PeProTech¹ | + | - |
| Human | Chinese Hamster Ovary (CHO) Cells | HCDS1 | + | - |
| Human | Yeast | HYPG4 | + | - |

| | | | | |
|---|---|---|---|---|
| ¹Available from PreproTech EC Ltd. of Rocky Hill, New Jersey | | | | |

Baculovirus was found to be a contaminant of baculovirus-expressed CCL21 prepared as described in Example 1. Baculovirus-expressed CCL21 was used to prepare a Western Blot, which was probed with an antibody that specifically recognizes the baculoviral protein gp64. As shown in Figure 8, gp64 was detected in baculovirus-expressed CCL21 preparations. In addition, lots of baculovirus-expressed CCL21 that showed anti-tumor activity had relatively high titers of live virus, while inactive lots had relatively lower titers of live virus (Table 2).

**Table 2. Viral Titer Correlates with Anti-Tumor Activity of Baculavirus-Expressed CCL21 Preparations**

| Lot | Concentration (mg/ml) | PFU/ml | Regimen (µg/dose) | PFU/dose | Total PFU delivered | Activity |
|---|---|---|---|---|---|---|
| HBDS4 (filtered) | 0.5 | <20 | 25 | <1 | <6 | - |
| HBDS4¹ | 0.5 | <30 | 25 | <1.5 | <9 | - |
| HBDS4² | 0 | 600 | 25 | 30 | 180 | - |
| HBMC1 | 0.5 | 1.0 X 10⁴ | 100 | 5.0 X 10² | 3.0 X 10³ | - |
| HBDS1 | 1.8 | 1.0 X 10⁶ | 25 | 1.4 X 10⁴ | 8.3 X 10⁴ | + /- |
| HEPG3 +bv³ | 0.5 | 1.3 X 10⁶ | 25 | 6.5 X 10⁴ | 3.9 X 10⁴ | + |
| HBDS4 | 0.8 | 5.0 X 10⁵ | 25 | 1.6 X 10⁴ | 9.00 X 10⁴ | + |
| HBPG1 | 1.8 | 2.7 X 10⁷ | 25 | 3.8 X 10⁵ | 2.00 X 10⁶ | + |

| | | | | | | |
|---|---|---|---|---|---|---|
| ¹small fraction; ²large fraction; ³bv, baculovirus | | | | | | |

Figure 9 shows that intratumoral injection of purified live baculovirus, in the absence of CCL21, and at titers comparable to those seen in contaminated CCL21 preparations, inhibits tumor growth as effectively as the baculovirus-contaminated CCL21 preparations.

**Example 8. Baculovirus-Induced Cytotoxicity *In vitro***

Baculovirus were prepared as described in Example 1. Uninfected Sf9 cells were used as a control. Following centrifugation, both the supernatants and cellular pellets were recovered. For performance of the cytotoxicity assay, the samples were diluted 1:11 in growth media to an initial virus titer of 5 X 10⁶ pfu/ml.

A549 human epithelial lung cells were seeded in triplicate into 96-well tissue culture plates and incubated with serial dilutions of supernatant or cells from baculovirus-infected or uninfected Sf9 cells. After 24 hours, the media were removed, and the cells were washed extensively with fresh media. Cell viability was determined 24 to 48 hours later by crystal violet staining, which was quantified by spectroscopy.

As shown in Figure 10A, the cell pellet samples induced a greater cytotoxic response than the supernatant samples. Supernatant collected from uninfected cells did not induce cytotoxicity. Figure 10B shows that the cytotoxic response was significantly reduced when the baculovirus-expressed supernatant was filtered through a 0.2 µm filter (to remove contaminating baculovirus), similar to the loss of *in vivo* response- The disclosed *in vitro* cytotoxicity can be used to predict *in vivo* anti-cancer activity. See also Figure 13.

**Example 9. Baculovirus Activates Dendritic Cell Maturation**

Addition of wild type baculovirus to dendritic cell (DC) cultures induced their maturation, as evidenced by increased cell surface expression of activation markers. As shown in Figures 11A and 11B. baculovirus activates mouse bone marrow-derived DCs and human monocyte-derived DCs.

Mouse DCs were prepared from bone marrow according to standard methods. Briefly, bone marrow was isolated from female Balb/c or C57BI/6 mice, 6-8 weeks old, (Charles River Laboratories of Holister, California) and frozen (-80oC) in heat-inactivated fetal bovine serum supplemented with 10% cell-culture grade dimethyl sulfoxide (DMSO) at a density of 2 X 10⁷ cells/ml. Frozen cell aliquots were rapidly thawed and washed to remove DMSO. Cells were plated in 150 mm suspension culture dishes containing 20 ml supplemented RPMI media (Sigma-Aldrich of St. Louis, Missouri) containing 200 U/ml murine GM-CSF (PreproTech of Rocky Hill, New Jersey). On day 3 of culture, cells were again supplemented with murine GM-CSF, and on day 5, one-half of the culture volume was centrifuged to replace fresh medium containing GM-CSF. BMDC were harvested by gentle pipetting.

Baculovirus and other control materials were added to the media on day 6. Cells were incubated an additional 18 hours prior to analysis of cells or supernatants. BMDC were analyzed for cell surface markers by FACS and were characterized as immature on day 6 prior to addition of stimuli by detection of markers for DC immaturity, including CD11c, CD11b, H-2Kd, I-Ad(low), CD80(low), and CD86(low). Following overnight incubation with various stimuli, cells were washed and double-stained using anti-CD11c and anti-CD86 or anti-I-A antibodies and then analyzed by flow cytometry. Cells were gated on the live CD11c+ population. Stimulation is expressed as the mean fluorescence intensity (MFI) divided by MFI from stained cells treated only with GM-CSF. Figure 11A shows that the expression of the DC activation marker CD86 and MHC class 11 (detected using anti-I-A antibodies) was increased in response to baculovirus. The levels of CD80 and CD40 were similarly elevated in response to baculovirus.

Human DC were derived from peripheral blood monocytes purified from the buffy coats of healthy volunteers by using anti-CD14 antibody-coated magnetic beads (Miltenyi Biotec of Auburn, California). Immature DC were harvested after 3-4 days of culture with interleukin-4 and GM-CSF (each 1000 U/ml; available from PreproTech of Rocky Hill, New Jersey). Cultures were routinely >90% CD1a positive by FACS (Pharmingen of San Diego, California). FACS analysis of DC activation markers was assessed by gating on live CD1a+ cells. Figure 11B shows that baculovirus induced elevated levels of GD86 and HLA-DR++.

**Example 10. Baculovirus Activates CTL Induction *In vivo***

Immunization of mice with baculovirus and a soluble protein antigen (HIV p24) induced a robust, antigen-specific CTL response. Spleens from immunized mice were harvested 2 weeks following the third immunization. Individual spleens were combined such that 5 spleens were included in each sample. Spleen cells from immunized mice were cultured in a 24-well dish at 5 X 10⁶ cells per well, Of these cells, 1 x 10⁶ cells were sensitized with: (1) a synthetic p7g peptide, which is an H-2Kd restricted CTL epitope corresponding to amino acids 199-208 of HIV-1SF2p24gag; and (2) a pGagb peptides, which is an H-2Db restricted CTL epitope corresponding to amino acids 390-398 of HIV-1_{SF2}p55gag. The peptides were used at a concentration of 10µM for 1 hour at 37°C. Splenocytes were then washed and co-cultured with the remaining 4 X 10⁶ untreated cells. The splenocytes were stimulated as a bulk culture in 2 ml of splenocyte culture medium: RPMI 1640 (Sigma-Aidrich of St. Louis, Missouri) with 100 mM L-glutamine (Gibco of Grand Island, New York) and α-Mem (Minimum Essential medium Alpha Medium with L-glutamine, deoxyrobonucleosides or ribonucleosides) (1:1), supplemented with 10% heat inactivated fetal calf serum (Hyclone of Logan, Utah), inactivated in a 56°C water bath for 30 minutes in 100 U/ml penicillin, 10 µg/ml streptomycin, 10 ml/L of 100mM sodium pyruvate and 50µM 2-mercaptoethanol. In addition, 5% Rat T-Stim IL2 (Rat T-Stim: Collaborative Biomedical Products of Bedford, Massachusetts) was used as a source of IL2 and was added to the culture media just before the cells were cultured.

After a stimulation period of 6-7 days, splenocytes were collected and used as effectors in a chromium release assay. Approximately 1 X 10⁶ SV/Balb or MC57 target cells were incubated in 200 µl of medium containing 50 µCi of 51 Cr and with the correct peptide p7g, or a mismatched cell-target pair as the negative control, at a concentration of 1µM for 60 minutes and washed. Effector cells were cultured with 5 x 10³ target cells at various effector to target ratios in 200 µl of culture medium in 96-well round or V-bottom tissue culture plates for 4 hours. The average counts per minute from duplicate wells was used to calculate percent specific release. Figure 12 shows that baculovirus induced cytolytic T cell responses.

**Example 11. Photochemical Inactivation of Baculovirus**

Two liter suspension cultures of *Trichoplusia ni* (Tn) cells are infected with baculovirus. Following incubation for 3 days at 28°C the infected cell suspension is harvested and clarified by centrifugation at 800 X *g* for 10 minutes. The titer of baculovirus in the harvested medium was determined by plaque assay in *Spodoptera frugiperda* (Sf) cells, for example as described by King & Possee (1992) The Baculovirus Expression System: A Laboratory Manual, Chapman & Hall, London.

A stock solution of trioxalen is prepared at a concentration of 0.2 mg/ml in dimethyl suffoxide (DMSO). Trioxalen is added to the infected cell suspension at a concentration of about 5-10 µg/ml and dispersed within the cell suspension by gentle shaking. The cell suspension is then poured into a seamless, stainless steel tray (e.g., about 1 cm in depth) and placed on a rotating platform. A long wavelength (365 nm, 6W) UV lamp is placed directly above the tray at a distance of 1 cm from the liquid surface. Exposure to UV illumination is allowed to proceed for about 15 minutes, or for a period sufficient for virus inactivation.

To assess virus inactivation, the trioxalon/UV inactivated samples are titrated on insect cells. For example, aliquots are taken from the cell suspension, serially diluted, and used to inoculate Sf9 cell cultures. The medium is changed at about 16 hours post inoculation to minimize DMSO-induced cytotoxicity. The cultures are examined microscopically to assess cellular pathology 7 days post inoculation and, if negative, are passaged twice more to confirm virus inactivation. The cultures are also examined to identify cellular cytotoxicity.

**Example 12. *In vitro* Assay Predicts *In vivo* Anti-Tumor Efficacy**

Various lots of CCL21 were tested both in a cytotoxicity assay and in an *in vivo* mouse tumor model, both of which are described below. Cytotoxicity activity coefficient is described below. Active and inactive lots were determined by analysis of tumor size at the end of the *in vivo* experiment.

**Induced PBMC Cytotoxic Assay**

The induced PBMC Cytotoxic Assay measures the cytotoxic (or cytostatic) response of PBMCs induced by certain activating substances such as cytokines (lL-2 or β-IFN) or baculovirus (BV) against an adherent target cell line (A549 or MG-63 cells), and uses a co-oulture technique of effector cells (PBMCs) and target cells. After an incubation period the target cell viability is quantified by Alamar blue staining. Components used in the assay include:
Growth Medium (GM): (Eagle's MEM with Earle's salts and 2.2 g/L sodium bicarbonate, Fetal Bovine Serum (FBS), L-Glutamine, penicillin, and streptomycin):

| | |
|---|---|
| MEM | 500 ml |
| FBS | 50 ml |
| L-Glutamine (200 mM) | 5 ml |
| Penicillin (10,000 U/ml)/streptomycin (10,000 µgml)/mix | 5 ml |

Growth / Assay Medium (GAM): (RPMI 1640 w/o pH indicator, Fetal Bovine Serum (FBS), L-Glutamine).

| | |
|---|---|
| RPMI 1640 | 500 ml |
| FBS | 50 ml |
| L-Glutamine (200 mM) | 5 ml |

PBMC Prep Medium: (RPMI 1640 w/o pH indicator, 0.5% BSAfractian V):

| | |
|---|---|
| RPMI 1640 | 500 ml |
| BSA (7.5% BSA soluflon) | 37 ml |

STV Solution: (Saline A, trypsin, versene (Na₄ EDTA)):.

| | |
|---|---|
| NaCl | 8.00 g |
| KCl | 0.40 g |
| D-glucose | 1.00 g |
| NaHCO₃ | 0.58 g |
| Trypsin 1:250 | 0.50 g |
| Na₄EDTA | 0.20 g |
| 0.5% phenol red | 0.9 ml |
| Glass distilled water | q.s, to 1.0L |

Phosphate Buffered Saline: (PBS, calcium-free and magnesiurri-free).

| | |
|---|---|
| KCl | 0.2 g |
| NaCl | 8.0 g |
| KH₂PO₄ | 0.2 g |
| Na₂HPO₄ | 1.14 g |
| Glass distilled water | q.s. to 1.0 L |

| | |
|---|---|
| Alamar Blue staining media RPMI 1640 with 10% Alamar blue (from Biosource International) | |

**A549 Cells**

A549 human lung carcinoma cells were obtained from American Type Culture Collection at passage 76 (ATCC CCL 185). Cells were expanded and stocks frozen preserved at low passage. A Master stock of A549 cells was frozen and preserved in liquid nitrogen.

**A549 Working Culture**

A549 cells grow as an adherent monolayer and must be detached with a trypsin solution for subculture. Media was removed from each T-175 flask. The monolayers were washed twice with 10 to 15 ml PBS. Three to 5 ml STV were added to each flask Each flask was then incubated for 3 to 4 minutes or until cells started to detach. Flasks were gently tapped to detach cells. Five ml of Growth Medium was added to each flask and cells were gently triturated with a pipette to prepare a single cell suspension. Cells were transferred to a 50 ml centrifuge tube containing fresh Growth medium and gently mixed. Various proportions (1:5, 1:10 or 1: 20, for 2, 3, and 4 day cultures respectively) of cell mixture were distributed into T175 culture flasks containing 40 ±/- 5 ml of fresh Growth Medium warmed to 37°C.

**MG-63 Cells**

The MG-63 human osteosarcoma cell line was obtained from the American Type Culture Collection (ATCC CRL-1427). A master stock of MG-63 cells was frozen and preserved in liquid nitrogen.

**MG-63 Working Culture**

Working Stock cultures were split every 3 or 4 days. Confluent monolayers were split 1:6 on a 3 day schedule and 1:8 on a 4 day schedule.

**Subculture procedure:**

MG-63 cells grow as an adherent monolayer and must be detached with a trypsin solution for subculture. Media is aspirated from each T-175 flask and the monolayer washed twice with 10 to 15 ml PBS. Four ± 0.1 ml STV was added to each flask and then flasks then incubated at 37° ±2°C, 5 ±1% CO₂, for 3 to 5 minutes until cells start to detach. Flasks were gently tapped to detach cells.

Cells were pipetted up and down in STV to obtain a uniform suspension and then transferred to a 50 ml centrifuge tube containing fresh Growth medium and gently mixed. Proportions (1:6 or 1:8) of cell mixture were dispensed into culture flasks containing 40 ±/-5 ml of fresh Growth medium warmed to 37°C.

**PBMC Cells**

PBMCs were obtained from unanimous donors.

**Preparation of A549 or MG-63 cells for PBMC Induced Cytotoxic Assay**

Media was removed from the A549 or MG-63 Working Culture T-175 flask. The monolayers were washed twice with 10 to 15 ml PBS. Three to 5 ml STV were added to each flask and the flasks were then incubated for 5 minutes or until cells started to detach. Flasks were gently tapped to detach cells. Five ml of Growth Medium were added to each flask and cells gently triturated with a pipette to prepare a single cell suspension. Cells were transferred to a 50 ml conical tube and an additional 25ml Growth Media was added. The tube was inverted to mix cells, yielding a cell concentrate. Cell density was determined in the cell concentrate. Briefly, a 1:3 dilution of the cell suspension was counted using a model Z1 Coulter counter. For A549 cells the lower threshold of the counter was set to 8 microns and for MG-63 cells set to 8 microns.

For A549 cells the cells were concentrated to 50,000 cells/ml in Growth Assay Medium. For MG-63 cells the cells were concentrated to 65,000 cells/ml in Growth Assay Medium. The total number of cells needed was calculated by multiplying the total volume of media needed by the seeding density. The volume of cell concentrate needed (C) was calculated by dividing the total number of cells needed by the cell density of concentrate. The volume of additional growth/assay media needed (GAM) was calculated by subtracting the volume of cell concentrate from the volume of media needed. The proper seeding density of target cells used for PBMC induced cytotoxic assay was determined by combining (C) and (GAM).

The cell suspension was prepared in a disposable Erlenmeyer flask or tissue culture glassware. Cells were added to assay plates within 15 minutes. One hundred µl of the suspension (5000 cells / well for A549 cells and 6500 cells *l* well for MG-63 cells) were added to each well, The plates were incubated with lids for 18 to 24 hours in a humidified 37° +2°C, 5 ±0.5% CO₂ incubator.

**Initial Sample Preparation and Serial dilutions in transfer plates**

**Initial Screening Assay**

In order to improve the chance of detecting inducers of PBMC cytotoxicity the samples were initially prepped at a relatively high concentration. The sample concentration was reduced in subsequent assays depending on sample activity.

**Low concentration CCL21 Samples:**

Low protein concentration samples of CCL21 (below 3000 µg /ml) were diluted to between 400 and 600 rg/ml with PBS (without calcium or magnesium). FBS and I-glutamine were added to make the sample 10% FBS and 1% I-glutamine. Samples were loaded into the 1^{st} well of a 96 well plate neat (240 µl volume). One:two serial dilutions were done in a 96 well transfer plate into PBS with 10% FBS and 1% I-glutamine.

Samples that started out at a high concentration (of protein, or activity, or cells, or virus) were prepped as described for low concentration protein samples or prepped into growth/ assay media directly. Samples were loaded into the 1^{st} well of a 96 well plate neat (240 µl volume). One:two serial dilutions were done in a 96 well transfer plate into growth assay media.

**Baculovirus samples:**

BV samples were diluted from 1:2 to 1:10 in growth / assay media (GAM) and serially diluted as described above into GAM.

β**-IFN or IL-2:**

Final vial samples of β-IFN or IL-2 were reconstituted in the proper diluent (saline or water for injection). Reconstituted β-IFN was at 0.25 mg/ml = 13.9µM. reconstituted IL-2 was at 1.1mg/ml = 64.7µM. A 200,000 IU/ml working stock of either cytokine was made by further diluting with growth assay media (GAM). The β-IFN lot IFN-01-001 was diluted 1: 35.75 with GAM. The IL-2 lot MLAPM006 was diluted 1:91.75 with GAM. subsequent dilutions to assay concentration were with GAM. IL-2 was diluted to 2000IU/ml for assay β-IFN was diluted to 2000 IU/ml for assay.

**High Concentration CCL21 Samples:**

CCL21 samples with concentrations above 3 mg /ml were diluted to assay concentration of 200 to 600 µg/ml with GAM.

**Transfer of sample dilutions to Target Cell (Assay) plates:**

The contents of the dilution plate were transferred to the assay (Cell) plate. The final sample concentration was ½ that of the original dilution plate. A propette with plate to plate transfer program for was used for the transfer. Assay plates were incubated while prepping the PBMCs.

**Toxicity Control plate for direct cytotoxicity of test samples to target cells:**

Two identical transfer (dilution) plates were established, and media transferred to 2 Cell plates. To Plate 1 50µl of PBMC prep was added for induced cytotoxicity measurements. To the 2^{nd} plate, 50µl PBMC prep media was added for measuring direct cytotoxicity of test samples.

**Isolating peripheral blood mononuclear cells (PBMC) from Human peripheral Blood**

A 1:10 dilution of bleach (3 liters) was prepared. All the tubes and pipettes contacting with blood were bleached over night The beaker was drained and all items discarded in a sharps or biohazard container. The caps were removed from the 50 ml tubes before handling the blood (to prevent contaminating the caps with blood). The blood was added to 250m1 disposable polycarbonate flask and diluted with an equal volume of HBSS. Approximately 17ml of ficoll-plaque solution per 50 ml polystyrene tube was added 30 minutes before adding the blood. The blood was layered on the top of the ficoll-plaque solution without disturbing the ficoll layer. Four ml blood/HBSS was added for each 3 ml ficoll-plaque. (i.e. 17ml ficoll and 23 ml blood/HBSS per 50ml tube).

Tubes were centrifuged at 1800 RPM (400 xg) for 30minutes at room temp. (Sorval GLC-2B centrifuge). As much of the top layer as possible was aspirated using a 25ml serological pipette, leaving about 5 ml on top of the second layer. A 5 ml serological pipette was used to remove the rest of the first layer. The second layer, containing the B and T lymphocytes, was collected using a sterile 5ml serological pipette and placed into a new 50ml tube. The collection was diluted with 3x the volume of RPMI with no additives. The resulting solution was centrifuged at 900 rpm (100 x g) for 15-20 minutes. (Wash 1). The supernatant was removed with a serological pipette and the cells resuspended in 40 ml RPMI and centrifugation was repeated at 900 rpm (Wash 2). The supernatant was removed with a serological pipette and the cells resuspended in 40 ml PBMC prep media (RPMI with 0.5% BSA). The total volume of the re-suspended cells was accurately recorded. The cells were counted with a Coulter counter to determine the cell density- a 1:5 dilution of cell concentrate in PBMC prep media was used for the count).

The re-suspension volume needed to attain proper cell density was determined. For the Cytotoxic Assay the desired density was 2 x 10⁶ cells /ml and for freezing of the PBMCs the desired density was 10 x 10⁶ cells/ml. The final re-suspension volume was calculated by multiplying cell density by total volume and then dividing the product by the final desired cell density (2 or 10 x 10⁶ cell/ml).

Centrifugation was repeated at 900rpm with the remainder of the re-suspended cells. (Wash3). The supernatant was removed and resuspended in final re-suspend volume. The cell density was confirmed with a Coulter counter (a 1:10 dilution of cell concentrate in PBMC prep media was used for the count).

**PBMC Prep for Assay**

For immediate use in assay, cells were resuspended in PBMC prep media. The cells were further diluted 1:2 in PBMC prep Media to a final cell density of 1 x 10⁶ cell / ml. The cells were added to Assay plates with target cells 50 µl/well (50000 PBMCs/well). PBMCs were added with a Multichannel pipette. Assay plates were incubated at 31°C and 5% CO₂ for 3 to 4 days.

**PBMC Prep for Freezing**

For freezing cells were resuspended in 90% FBS (heat inactivated) + 10% DMSO and frozen at a density of 10 x 10⁶ cells/ml. The cells were aliquoted in 2ml sterile cryotubes and frozen in isopropyl alcohol in cryo-freezing containers in -70°C freezer. The final cell density was confirmed with a Coulter counter.

**Thawing frozen PBMCs for Assay**

Frozen aliquots of PBMCs were quickly thawed in 37°C water bath. Cells in were resuspended in 13ml RPMI 1640 in a conical centrifuge tube and then centrifuged at 900 rpm (100 x g) for 15-20 minutes. The supernatant was removed with a serological pipette and the cells were resuspended in 13 ml PBMC prep media (RPMI with 0.5% BSA). Cells were counted with a Coulter counter to determine the cell density - a 1:5 dilution of cell concentrate in PBMC prep media was performed for the count).

The re-suspension volume needed to attain proper cell density was then determined For the Cytotoxic Assay the desired cell density was 2 x 10⁶ cells /ml. The final re-suspension volume was determined by muliplying the Cell density by the total volume and ten dividing the product by the final desired cell density (2 or 10 x 10⁶ cell/ml). Centrifugation was repeated at 900rpm with the remainder of the re-suspended cells. The supernatant was removed and the cells resuspended in final re-suspend volume.

The cell density was confirmed with a Coulter counter - a 1:10 dilution of cell concentrate in PBMC prep media was performed for the count). Cells were further diluted 1:2 in Growth Assay Media to a final cell density of 1 x 10⁶ cell / ml. Cells were then added to Assay plates with target cells 50 µl/well (50000 PBMCs/well). PBMCs were added with a Multichannel pipette. Assay plates were incubated at 37°C and 5% CO₂ for 3 to 4 days.

**Staining of Assay plate with Alamar Blue**

After 3 to 4 days of incubation, the media was aspirated from the assay plates. One hundred µl/well Alamar Blue staining media (10% Alamar Blue in RPMI with 0.5% BSA) was added, and the plates incubated for 2 to 3 hours. The response was measured using either a spectrophotometer (Absorbance at 570nm - 630nm) or flourometrically (excitation at 530nm and Emission at 590nm).

**Assay Activity**The assay activity was defined as the reference well response divided by the sample well with PBMC response. In most cases the reference well was the no PBMC control well for the sample at the same sample concentration as the PBMC well, usually at the highest sample concentration (the 1st well in the dilution series). In some instances when there was insufficient sample to run a no PBMC control plate the reference well is a PBS/growth media well with or without PBMCs.

When enough sample was present for a no PBMC control plate, the activity was set as equal to the Direct Cytotox response / Induced Cytotox response. As shown in Figure 14, *in vitro* data correlates with *in vivo* data.

**Example 13. Anti.gp64 Monoclonal Antibodies Blocks Baculovirus Tumor Cell Killing**

Starting material of baculovirus (CΔ3) was treated with anti-gp64. Briefly, 1-500 µg of purified mouse anti-gp64 monoclonal antibody (clone 1.3A provided by Dr. Donald Jarvis, U. of WY) or Ig control was mixed with various infectious units of recombinant baculovirus in a controlled volume. The binding of antibody to virus was conducted at room temperature, in the dark for approximately 15 hours. Samples were refrigerated for 1-2 days prior to plaque or cytotoxicity assay. Samples were tested in the cytotoxicity assay as described above. See Figure 15.

**Example 14. Inactivated Baculovirus Induces PBMC-Medlated Tumor Cell Killing *In vitro***

Starting material of baculovirus (CΔ3) was inactivated by treatment with trioxalen and UV light. Baculovirus was photo-inactivated by psoralen and ultra-violet light (Weightman, S. A. and Banks, M. J. Virol. Met. 81:179-182 (1999)). Insect cell culture media containing baculovirus was transferred (3 mL, approximately 6E7 pfu) to a 6-well cell culture plate (Costar). A stock solution of 4,5',8-trimethyl psoralen (trioxalen) was prepared at 2 mg/mL in dimethyl sulfoxide (DMSO) and added to each well to a final concentration of 100 µg/mL The culture dish was then exposed to ultra-violet (365 nm) light for 15 minutes by placing a hand-held lamp (Model UVL-56, UVP, Upland, CA) 1.5 cm over the plate. Control samples included DMSO with no psoralen, as well as a non-irradiated control with psoralen. After irradiation, media was transferred to a 10 MWCO dialysis cartridge (Slidalyzer, Pierce) and dialysed against phosphate-buffered saline. All samples were assayed for plaque-forming activity on Sf-9 cells. Samples were tested in the cytotoxicity assay as described above, and results are set forth in Figure 16.

**Example 15. Tumor Cells are the Principle Target for Baculovirus**

Intact monolayers of A549 target cells were treated with baculovirus for 3 hours and then washed 3 times with growth assay media. At the same time effector cells (PBMSc) were also treated with baculovirus for 3 hours and washed 3 times. Baculovirus treated and untreated target and effector cells were combined as follows: 1) Untreated target cells alone; 2) Untreated target and effector cells; 3) Baculovirus treated target cells and untreated PBMCs; and 4) Untreated target cells and baculovirus treated PBMCs.

After 4 days incubation the target cell viability was measured as described in SOP. The cell viability measurement, as determined by Alamar Blue fluorescence, was the mean of 16 wells per group (experiment was performed in a 96 well format). Results are set forth in Figure 17.

**Example 16. Tumor Growth inhibition in an Animal Model of Lung Cancer Using Baculovirus**

C57BL/6 mice at 8-10 weeks of age were allowed to acclimate for a minimum of 7 days prior to inoculation with tumor cells. Mice were inoculated s.c. at the right flank with 2 X 10⁵ early passage (<10 passages) 3LL-HM tumor cells. Tumor size was measured twice per week. When tumors reached 50-100 mm³ (typically 7 days after tumor inoculation), mice were randomized into groups and received their first dose of Baculovirus intratumorally on the same day. The dosing schedule was qd X 6 days. The mice were placed in four groups as follows: 1) Albumin negative control 25 µg/dose (0.5 mg/ml); 2) Live virus positive control (titer about 1X10⁷/ml); 3) live virus negative control (titer about 800/ml); and 4) inactivated virus (titer about 800/ml). Tumors were measured twice a week for up to four weeks. When tumor volume reached 2500 mm³ or when any of the following symptoms were seen the mice were sacrificed. The symptoms were body weight loss is more than 20%, tumor ulceration areas is more than 30% of tumor surface area or less than 30% of tumor surface but has openings, bleeding or discharging, server difficulty in breath, or moribund.

As shown in Figure 18 intratumoral administration of Baculovirus resulted in growth delay of 3LL tumors.

**Example 17. Protection from Infectious Agents In Vitro and In Vivo Using Baculovirus**

Live baculovirus has been shown to induce interferons (IFN) from murine and human cell lines and induces *in vivo* protection of mice from encephalomyocarditis virus infection. However, inactivation of the baculovirus by UV, for example, eliminates both infectivity and IFN-inducing activity. (Gronowski et al., J Virology, Dec. 1999, p. 9944-9951 Vol. 73, No. 12).

To study the effect of baculovirus on infectious agents, cells were challenged *in vivo* and *in vitro* with Vesicular Stomatitis Virus (VSV). As shown in Figure 19, cells or medium treated with Sf9 and baculovirus or with Sf9 and baculovirus media provided maximal protection against VSV *in vitro.* As shown in Figure 19, inactivated baculovirus protects *in vivo* and *in vitro* against pathogenic viral challenge.

**Example 18: Bystander Effect**

To investigate whether the inhibition of tumor cells is only direct (i.e. that each cell must be specifically targeted by a non-pathogenic virus) infected tumor cells and non-infected tumor cells were mixed in different proportions.

A549 or MG-63 cell lines or PBMCs, were exposed to an activating dose of baculovirus for 3 to 5 hours. After exposure to baculovirus the excess or non-binding virus was washed off from the responder cells. The washed BV treated cells were then mixed with non-treated responder cells in various proportions. For PBMCs the mixture of BV treated and non-treated cells were added to non-treated A549 or MG-63 target cells. Likewise, the mixture of BV treated A549 or MG-63 target cells were added to non-treated PBMCs. Non-BV treated cells were physically handled the same as BV treated cells.

PBMCs were treated with BV only while the cells were kept in suspension while shaking in a CO₂ incubator. The cells were washed by centrifugation, decanted and re-suspended (3 times) followed by mixing with non-treated PBMCs. The A549 and MG-63 target cells were treated with BV in 2 ways. Either the intact monolayer was treated with BV followed by gentle washing in place with fresh growth media, then non-treated target cells were added to the assay plates, or while in suspension as described for the PBMCs.

lf the Baculovirus was removed from the A549 and MG-63 target cells for an extended time before the addition of PBMCs there was a rapid loss of the cytotoxic response in the cytoxicity assay. By 20 hours after removal of the baculovirus the cytotoxic response was entirely gone with the A549 cells and mostly gone for the MG-63 cells. (Data not shown)

Washing of the baculovirus treated target cell monolayer in place, then mixing with non-treated cells and immediate addition of PBMCs results in a bystander effect for both A549 and MG-63 target cells.

BV treatment of PBMCs or A549 cells in suspension followed by centrifugal washing (3 cycles of centrifugation, decant, re-suspend) effectively eliminated the cytotoxic response.

BV treatment and centrifugal washing of MG-63 cells was less effective in removing the cytotoxic response with MG-63 target cells,

PBMCs treated with BV in suspension while shaking followed by centrifugal washing seemed to result in a significant non-specific (in wells with 0% BV treated cells) stimulation of the cytotoxic response against MG-63 target cells but not A549 calls.

As seen in Figure 20, when the total cell volume was 20% infected cells and 80% non-infected cells, cell death was observed at the maximal response. Therefore, the non-infected cells were killed by what is known by the bystander effect (i.e. being in the vicinity of targeted cells promotes the cell death of non-targeted (infected) cells).

### References

The references listed below as well as all references cited in the specification are incorporated herein by reference to the extent that they supplement, explain, provide a background for or teach methodology, techniques and/or compositions employed herein.
Ausubel F, ed. (1995) Short Protocols in Molecular Biology, 3rd ed. Wiley, New York.
Baggiolini M, Dewald B & Moser B (1997). Human chemokines: an update. Annu Rev Immunol 15:675-705.
Berkow R, Beers MH & Fletcher AJ (1997) The Merck Manual of Medical Information, Home ed. Merck Research Laboratories, Whitehouse Station, New Jersey.
Bodanszky M (1993) Principles of Peptide Synthesis, 2nd rev. ed. Springer-Verlag, Berlin / New York.
Boyce FM & Bucher NL (1996). Baculovirus-mediated gene transfer into mammalian cells. Proc Natl Acad Sci USA 93:2348-2352.
Bronte V (2001). Genetic vaccination for the active immunotherapy of cancer. Curr Gene Ther 1:53-100.
Carbonell LF, Klowden MJ & Miller LK (1985). Baculovirus-mediated expression of bacterial genes in dipteran and mammalian cells. J Virol 56:153-160.
Carbonell LF & Miller LK (1987). Baculovirus interaction with nontarget organisms: a virus-borne reporter gene is not expressed in two mammalian cell lines. Appl Environ Microbiol 53:1412-1417.
Cotten M, Wagner E, Zatloukal K, Phillips S, Curiel DT & Birnstiel ML (1992). High-efficiency receptor-mediated delivery of small and large (48 kilobase gene constructs) using the endosome-disruption activity of defective or chemically inactivated adenovirus particles. Proc Natl Acad Sci USA 89:6094-6098.
Ebadi MS (1998) CRC Desk Reference of Clinical Pharmacology. CRC Press, Boca Raton, Florida.
Fearon DT (1997) Seeking Wisdom in Innate Immurnity. Nature 388:323-324.
Fearon DT & Locksley RM (1996) The Instructive Role of Innate Immunity in the Acquired Immune Response. Science 272:50-53.
Fehniger TA, Cooper MA & Caligiuri MA (2002). Interleukin-2 and interleukin-15: immunotherapy for cancer. Cytokine Growth Factor Rev 13:169-183.
Fields B, Knipe D & Howley P (1996). Virology, Vol 1, 3rd ed. Lippincott-Rave Publishers, Philadelphia, Pennsylvania.
Glover DM & Hames BD (1995) DNA Cloning: A Practical Approach, 2nd ed. IRL Press at Oxford University Press, Oxford / New York.
Goodman LS, Gilman A, Hardman JG, Gilman AG & Limbird LE (1996) Goodman & Gilman's the Pharmacological Basis of Therapeutics, 9th ed. McGraw-Hill Health Professions Division, New York.
Groner A, Granados RR & Burand JP (1984). Interaction of Autographa californica nuclear polyhedrosis virus with two nonpermissive cell lines. Intervirology 21:203-209.
Gronowski AM, Hilbert DM, Sheehan KC, Garotta G & Schreiber RD (1999). Baculovirus stimulates antiviral effects in mammalian cells. J Virol 73:9944-9951,
Henzler HJ & Kaiser K (1998). Avoiding viral contamination in biotechnological and pharmaceutical processes. Nat Biotechnol 16:1077-1079.
Hofmann C, Sandig V, Jennings G, Rudolph M, Schlag P & Strauss M (1995). Efficient gene transfer into human hepatocytes by baculovirus vectors. Proc Natl Acad Sci USA 92:10099-10103.
Homey B, Muller A & Zlotnik A (2002). Chemokines: agents for the immunotherapy of cancer Nat Rev Immunol 2:175-184.
Hwang GS, Kim JK & Choi BS (1996). The solution structure of a psoralen crosslinked DNA duplex by NMR and relaxation matrix refinement. Biochem Biophys Res Commun 219:191-197.
Janeway CA, Jr. (1989) Approaching the Asymptote? Evolution and Revolution in Immunology. Cold Spring Harb Symp Quant Biol 54:1-13.
Jung S & Littman DR (1999). Chemokine receptors in lymphoid organ homeostasis. Curr Opin Immunol 11:319-325.
Katzung BG (2001) Basic & Clinical Pharmacology, 8th ed. Lange Medical Books I McGraw-Hill Medical Pub. Division, New York.
King L & Possee R (1992). The Baculovirus Expression System: A Laboratory Manual, Chapman & Hall, London.
Kool M & Vlak JM (1993). The structural and functional organization of the Autographa californica nuclear polyhedrosis virus genome. Arch Virol 130:1-16.
Manome Y, Abe M, Hagen MF, Fine HA & Kufe DW (1994). Enhancer sequences of the DF3 gene regulate expression of the herpes simplex virus thymidine kinase gene and confer sensitivity of human breast cancer cells to gancilovir. Cancer Res 54:5408-5413.
Marx G, Mou X, Freed R, Ben-Hur E, Yang C & Horowitz B (1996). Protecting fibrinogen with rutin during UVC irradiation for viral inactivation. Photochem Photobiol 63:541-546.
McIntosh AH & Shamy R (1980). Biological studies of a baculovirus in a mammalian cell line. Intervirology 13:331-341.
McLachlin JR, Yang S & Miller LK (1998). A baculovirus mutant defective in PKIP, a protein which interacts with a virus-encoded protein kinase. Virology 246:379-391.
Parmiani G, Castelli C, Dalerba P, Mortarini R; Rivoltini L, Marincola FM & Anichini A (2002). Cancer immunotherapy with peptide-based vaccines: what have we achieved? Where are we going? J Natl Cancer Inst 94:805-818.
Partington S, Yu H, Lu A & Carstens EB (1990). Isolation of temperature sensitive mutants of Autographa californica nuclear polyhedrosis virus; phenotype characterization of baculovirus mutants defective in very late gene expression. Virology 175:91-102.
Pathak M (1974). Phytophotdermatitis. In Sunlight and Man, M Pathak & T Fitzpatrick, eds. University of Tokyo Press, Tokyo.
PCT International Publication No. WO 00/38706
Pearson MN, Russell RL, Rohrmann GF & Beaudreau GS (1988). p39, a major baculovirus structural protein: immunocytochemical characterization and genetic location. Virology 167:407-413.
Remington JP, Osol A, Anderson JT, Hoover JE & Skolaut MW (1975) Remington's Pharmaceutical Sciences, 15th ed. Mack Pub. Co., Easton, Pennsylvania.
Rohrmann GF (1992). Baculovirus structural proteins. J Gen Virol 73 ( Pt 4):749-761,
Romagnani S (1992) Induction of Th1 and Th2 Responses: A Key Role for the 'Natural' Immune Response? Immunol Today 13:379-381.
Rueda P, Fominaya J, Langeveld JP, Bruschke C, Vela C & Casal JI (2000). Effect of different baculovirus inactivation procedures on the integrity and immunogenicity of porcine parvovirus-like particles. Vaccine 19:726-734.
Sattzman WM & Fung LK (1997). Polymeric implants for cancer chemotherapy. Adv Drug Deliv Rev 26:209-230.
Sambrook et al., eds. (1989) Molecular Cloning: A Laboratory Manual. Cold Springs Harbor Laboratory Press, Cold Spring Harbor, New York.
Schneider CH & Eberle AN (1993) Peptides, 1992: Proceedings of the Twenty-Second European Peptide Symposium, September 13-19, 1992, Interlaken, Switzerland. Escom, Leiden.
Schreiber RD, Hicks LJ, Celada A, Buchmeier NA & Gray PW (1985). Monoclonal antibodies to murine gamma-interferon which differentially modulate macrophage activation and antiviral activity. J Immunol 134:1609-1618.
Schroder E & Lübke K (1965) The Peptides. Academic Press, New York.
Silhavy TJ, Berman ML & Enquist LW (1984) Experiments with Gene Fusions. Cold Spring Harbor Laboratory, Cold Spring Harbor, New York.
Speight TM, Holford NHG & Avery GS (1997) Avery's Drug Treatment: A Guide to the Properties, Choice. Therapeutic Use and Economic Value of Drugs in Disease Management, 4th / ed. Adis International, Auckland / Philadelphia, Pennsylvania.
Summers MD & Smith GE (1978). Baculovirus structural polypeptides. Virology 84:390-402.
Thiem SM & Miller LK (1989). Identification, sequence, and transcriptional mapping of the major capsid protein gene of the baculovirus Autographa californica nuclear polyhedrosis virus, J virol 63:2008-2018.
Tjia ST, zu Altenschildesche GM & Doerfler W (1983). Autographa californica nuclear polyhedrosis virus (AcNPV) DNA does not persist in mass cultures of mammalian cells. Virology 125:107-117.
Tweeten K, Bulla L & Consigli R (1980). Characterization of an extremely basic protein derived from granulosis-virus nucleocapsids. J Virol 33:866-876.
U.S. Patent Application Publication No. 2002/0015945
U.S. Patent No. 4,551,482
U.S. Patent No. 4,764,369
U.S. Patent No. 4,820,805
U.S. Patent No. 5,106,699
U.S. Patent No. 5,405,770
U.S. Patent No. 5,418,130
U.S. Patent No. 5,490,840
U.S. Patent No. 5,510,103
U.S. Patent No. 5,651,991
U.S. Patent No. 5,688,931
U.S. Patent No. 5,714,166
U.S. Patent No. 5,750,383
U.S. Patent No. 5,786,387
U.S. Patent No. 5,855,900
U.S. Patent No. 5,858,410
U.S. Patent No. 5,922,356
U.S. Patent No. 5,922,545
U.S, Patent No. 5,994,392
U.S. Patent No. 6,001,806
U.S_ Patent No. 6,090,925
U.S. Patent No. 6,106,866
U.S. Patent No. 6,120,787
U.S, Patent No. 6,190,700
U.S. Patent No. 6,194,192
U,S. Patent No. 6,221,958
U.S. Patent No. 6,238,704
U.S. Patent No. 6,238,705
U.S. Patent No. 6,245,740
U.S. Patent No. 6,248,514
U,S. Patent No. 6,262,127
U.S. Patent No. 6,267,981
U.S. Patent No. 6,287,587
U.S. Patent No- 6,296,842
U,S. Patent No. 6,312,713
U.S. Patent No. 6,335,035
U.S. Patent No. 6,379,958
Vialard JE & Richardson CD (1993). The 1,629-nucleotide open reading frame located downstream of the Autographa californica nuclear polyhedrosis virus polyhedrin gene encodes a nucleocapsid-associated phosphoprotein. J Viral 67:5859-5866,
Volkman L & Goldsmith P (1983). In vitro survey of Autographa californica nuclear polyhedrosis virus interaction with nontarget vertebrate host cells. Appl Environ Microbiol 45:1085-1093.
Weightman SA & Banks M (1999). Photochemical inactivation of recombinant baculovirus. J Viral Methods 81:179-182.
Wilson ME, Mainprize TH, Friesen PD & Miller LK (1987). Location, transcription, and sequence of a baculovirus gene encoding a small arginine-rich polypeptide. J Viro/ 61:661-666.

It will be understood that various details of the invention can be changed without departing from the scope of the invention. Furthermore, the foregoing description is for the purpose of illustration only, and not for the purpose of limitation--the invention being defined by the claims appended hereto.
The following represent further embodiments of the present invention:
1. A method of inducing an immune response in an animal comprising administering to said animal an amount of a composition comprising an inactive non- pathogenic virus effective to induce an immune response in said animal.
2. The method of embodiment 1, wherein the non-pathogenic virus is an insect- specific virus.
3. The method of embodiment 2, wherein the insect-specific virus is a virus of Baculaviridae family.
4. The method of embodiment 1, wherein said composition is administered intratumorally, peritumorally, intralesionally, perilesionally, or combinations thereof.
5. The method of embodiment 1, wherein the inactive non-pathogenic virus comprises a viral particle, or a viral component.
6. The method of embodiment 5, wherein the viral component is gp64.
7. The method of embodiment 1, wherein said immune response protects against infectious disease.
8. The method of embodiment 1, wherein said immune response protects against cancer.
9. The method of embodiment 1, wherein said immune response induces a T-cell memory response.
10. The method of embodiment 1 wherein said immune response promotes dendritic cell maturation.
11. The method of embodiment 7, wherein said infectious disease is a virus, fungi or bacteria.
12. A method of causing cell death in a cell comprising administering a composition comprising an amount of a non-pathogenic virus to said cell effective to cause cell death in said cell.
13. The method of embodiment 12, wherein the non-pathogenic virus is an insect- specific virus.
14. The method of embodiment 12, wherein the composition comprising a non- pathogenic virus is effective at a concentration of less than about 500,000 PFU or about 500,000 PFU Equivalents to cause cell death in greater than about 50% of the contacted cells in an in vitro assay.
15. The method of embodiment 13, wherein the insect-specific virus is a virus of Baculaviridae family.
16. The method of embodiment 15, wherein the virus of Baculaviridae family is a granulosis virus or a nucleopolyhedrosis virus.
17. The method of embodiment 16, wherein the nucleopolyhedrosis virus is Autographa californica nucleopolyhedrosis virus.
18. The method of embodiment 12, wherein said cell is a cancer cell.
19. The method of embodiment 18, wherein said cancer is lung, breast, prostate, colon, gastric, pancreatic, renal, or skin cancer
20. The method of embodiment 12, wherein said non-pathogenic virus is an inactivated virus, a viral particle, a virosome, a Virus Like Particle, a viral occlusion body, or a viral component.
21. The method of embodiment 20, wherein said viral component is at least two components of the virus, wherein said components of the virus are selected from the group consisting of a peptide, protein, nucleic acid, lipid or carbohydrate.
22. The method of embodiment 12, wherein the non-pathogenic virus comprises a membrane protein of a non-pathogenic virus.
23. The method of embodiment 22, wherein the membrane protein is gp64.
24. The method of embodiment 12, wherein the non-pathogenic virus is an inactivated virus.
25. The method of embodiment 24, wherein said inactivation was chemical inactivation, UV inactivation, radiological inactivation, or genetic inactivation.
26. The method of embodiment 24 wherein said inactivation was psoralen- inactivation, UV-inactivation, or a combination thereof.
27. The method of embodiment 12, wherein the composition comprising a non- pathogenic virus consists essentially of a virus of Baculaviridae family.
28. The method of embodiment 12, wherein the composition is co-administered with another agent, wherein said agent is a chemotherapeutic, an anti-cancer drug, a vaccine, or combinations thereof.
29. The method of embodiment 12 wherein the composition is administered with a second composition, said second composition comprising an antigen and an adjuvant.
30. The method of embodiment 28 or 29, wherein said composition and agent, or said composition and second composition, are co-administered simultaneously.
31. A method of eliciting a CTL response in an animal comprising administering a composition comprising an amount of a non-pathogenic virus to the animal effective to elicit a CTL response in the animal, wherein said non-pathogenic virus is an insect-specific virus.
32. The method of embodiment 31, wherein said composition comprising a non- pathogenic virus is effective at a concentration of less than about 500,000 PFU or about 500,000 PFU Equivalents to elicit a CTL response in greater than about 50% of the contacted cells in an in vitro assay.
33. The method of embodiment 31, wherein the animal is a human.
34. The method of embodiment 31, wherein said insect-specific virus is a virus of Baculaviridae family.
35. A method of inhibiting tumor growth in an animal comprising administering to said animal an amount of a composition comprising a non- pathogenic insect-specific virus effective to inhibit tumor growth in said animal.
36. The method of embodiment 35, wherein said composition comprising a non- pathogenic virus is effective at a concentration of less than about 500,000 PFU or about 500,000 PFU Equivalents to prevent cell growth in greater than about 50% of the contacted cells in an in vitro assay.
37. The method of embodiment 36, wherein said in vitro assay is cell growth in soft agar.
38. The method of embodiment 35, wherein the composition is administered intratumorally and/or peritumorally.
39. The method of embodiment 35, wherein the animal is a mammal.
40. The method of embodiment 39, wherein the mammal is a human or a mouse.
41. The method of embodiment 35, wherein the composition is administered in multiple doses.
42. The method of embodiment 35, wherein the non-pathogenic virus is an inactivated virus, a viral particle, a virosome, a Virus Like Particle, a viral occlusion body, or a viral component.
43. The method of embodiment 42, wherein the viral component comprises at least two viral proteins.
44. The method of embodiment 43, wherein the at least two viral proteins comprise gp64.
45. The method of embodiment 42, wherein the viral component is gp64.
46. The method of embodiment 42, wherein the viral component comprises one or more of a nucleic acid, a lipid or a carbohydrate.
47. The method of embodiment 35, wherein the non-pathogenic virus is an inactivated virus.
48. The method of embodiment 47, wherein the inactivation was heat- inactivation, chemical inactivation, or UV inactivation.
49. The method of embodiment 47 wherein said inactivation was psoralen inactivation, UV inactivation, or a combination thereof.
50. A method of treating cancer in an animal comprising administering to said animal an amount of a composition comprising a non-pathogenic virus effective to ameliorate cancer symptoms in said animal.
51. The method of embodiment 50, wherein the composition comprising a non- pathogenic virus is effective at a concentration of less than about 500,000 PFU or about 500,000 PFU Equivalents to prevent cell growth in greater than about 50% of the contacted cells in an in vitro assay.
52. The method of embodiment 50, wherein the non-pathogenic virus is an insect- specific virus.
53. The method of embodiment 52, wherein the insect-specific virus is a virus of Baculaviridae family.
54. A method of inhibiting cancer metastasis in an animal comprising administering to said animal an amount of a composition comprising a non- pathogenic virus effective to inhibit cancer metastasis in said animal.
55. The method of embodiment 54, wherein the composition comprising a non- pathogenic virus is effective at a concentration of less than about 500,000 PFU or about 500,000 PFU Equivalents to prevent cell growth in greater than about 50% of the contacted cells in an in vitro assay.
56. The method of embodiment 54, wherein the non-pathogenic virus is an insect- specific virus.
57. The method of embodiment 56, wherein the insect-specific virus is a virus of Baculaviridae family.
58. A method of imparting resistance to cancer re-challenge in an animal comprising administering to said animal a composition comprising a non-pathogenic virus effective to inhibit cancer re-challenge in the animal.
59. The method of embodiment 58, wherein the non-pathogenic virus is an insect- specific virus.
60. The method of embodiment 59, wherein the insect-specific virus is a virus of Baculaviridae family.
61. The method of embodiment 58, wherein the animal is a human or a mouse.
62. A method of inhibiting a non-neoplastic proliferative disorder in an animal comprising administering to said animal an amount of a composition comprising a non-pathogenic virus effective to inhibit a non-neoplastic disorder in the animal.
63. The method of embodiment 62, wherein the composition is administered intralesionally, perilesionally, or combinations thereof.
64. The method embodiment 62, wherein the animal is a mammal.
65. The method of embodiment 64, wherein the animal is a human.
66. A method of inhibiting hyperplasia in an animal comprising administering to said animal an amount of a composition comprising a non- pathogenic virus effective to inhibit hyperplasia in said animal.
67. The method of embodiment 66, wherein the non-pathogenic virus is an insect- specific virus.
68. The method of embodiment 66, wherein the insect-specific virus is a virus of Baculaviridae family.
69. The method of embodiment 66, wherein the animal is a human or a mouse.
70. The method of embodiment 66, wherein the composition is administered intratumorally, peritumorally, intralesionally, perilesionally, or combinations thereof.
71. A method of inhibiting metaplasia in an animal comprising administering to said animal an amount of a composition comprising a non- pathogenic virus effective to inhibit metaplasia in said animal.
72. The method of embodiment 71, wherein the non-pathogenic virus is an insect- specific virus.
73. The method of embodiment 72, wherein the insect-specific virus is a virus of Baculaviridae family.
74. The method of embodiment 71, wherein the animal is a human or a mouse.
75. The method of embodiment 71, wherein the composition is administered via intratumorally, peritumorally, intralesionally, perilesionally, or combinations thereof.
76. A method of inhibiting one or more symptoms of cancer in an individual in need thereof comprising administering to said individual an amount of a composition comprising an amount of a non-pathogenic virus effective to inhibit one or more symptoms of cancer in said individual.
77. The method of embodiment 76, wherein the non-pathogenic virus is an insect- specific virus.
78. The method of embodiment 76, wherein the insect-specific virus is a virus of Baculaviridae family.
79. The method of embodiment 76, wherein the composition is administered intratumorally, peritumorally, intralesionally, perilesionally, or combinations thereof.
80. The method of embodiment 76, wherein the non-pathogenic virus comprises an inactivated virus, viral particle, or viral component.
81. The method of embodiment 80, wherein the viral component is gp64.
82. The method of embodiment 76, wherein the one or more symptoms of cancer are tumor growth, abnormal cell growth, metastasis, angiogenesis, cell death or cell invasiveness.
83. The method of embodiment 76, wherein the one or more symptoms of cancer are weight loss, bleeding, difficulty in breathing, bone fractures, compromised immune system or fatigue.
84. A method of protecting an animal from an infectious disease comprising administering to said animal an amount of a composition comprising an inactive non-pathogenic virus effective to protect said animal from an infectious disease.
85. The method of embodiment 84 wherein said non-pathogenic virus is an insect- specific virus.
86. The method of embodiment 85 wherein said insect-specific virus is a virus of Baculaviridae family.
87. The method of embodiment 86, wherein the virus of Baculaviridae family is a granulosis virus or a nucleopolyhedrosis virus.
88. The method of embodiment 87, wherein the nucleopolyhedrosis virus is Autographa califomica nucleopolyhedrosis virus.
89. The method of embodiment 84 wherein said inactivation was one or more of genetic inactivation, chemical inactivation, photochemical inactivation, UV-light inactivation, heat inactivation, or radiological inactivation.
90. The method of embodiment 89 wherein said genetic inactivation is a temperature sensitive mutant.
91. A method of causing cell death in a population of cells comprising contacting a composition comprising an amount of a non-pathogenic virus to a portion of said population of cells effective to cause cell death in said population of cells.
92. The method of embodiment 91, wherein the non-pathogenic virus is an insect- specific virus.
93. The method of embodiment 91 wherein said portion of said population of cells is no more than about 30% of said population.
94. The method of embodiment 91 wherein said portion of said population of cells is no more than about 20% of said population.
95. The method of embodiment 92, wherein the insect-specific virus is a virus of Baculaviridae family.
96. The method of embodiment 95, wherein the virus of Baculaviridae family is a granulosis virus or a nucleopolyhedrosis virus.
97. The method of embodiment 96, wherein the nucleopolyhedrosis virus is Autographa califomica nucleopolyhedrosis virus.
98. The method of embodiment 91, wherein said non-pathogenic virus is an inactivated virus, a viral particle, a virosome, a Virus Like Particle, a viral occlusion body, or a viral component.
99. The method of embodiment 91 wherein the population of cells comprises peripheral blood cells, tumor cells, NK cells or macrophages.
100. A method of treating a disease in a subject in need thereof comprising: (a) inactivating a non-pathogenic virus, wherein said nonpathogenic virus is inactivated by adding trioxalen to the non-pathogenic virus at a concentration between about 5-10 pg/ml and illuminating said non-pathogenic virus with UV at about 365 nm and about 6W for about 15 minutes; (b) formulating said inactivated non-pathogenic virus into a pharmaceutical composition; and (c) administering said pharmaceutical composition to the subject.
101. The method of embodiment 100, wherein the insect-specific virus is a virus of Baculaviridae family.
102. The method of embodiment 100 wherein the disease is cancer or an infectious disease.
103. A method of predicting in vivo anti-tumor activity of a compound comprising: a) contacting the compound with tumor cells and peripheral blood mononuclear cells ; and b) measuring cell death of said tumor cells ; wherein compounds that cause cell death of contacted tumor cells are predicted in be active in vivo.
104. The method of embodiment 103 wherein said compound is an inactivated virus, a viral particle, a virosome, a Virus Like Particle, a viral occlusion body, or a viral component.
105. The method of embodiment 103 wherein said compound is derived from the baculoviridae family.
106. The method of embodiment 103 wherein said compound is an insect specific virus.
107. The method of embodiment 103, wherein said tumor cells are A549 cells, 3LL- HM cells, 4T1 cells, MT901 cells, MAT Blll cells, B16 melanoma cells or MG-63 cells.
108. A method of inhibiting an infectious disease in an animal comprising administering to said animal an amount of a composition comprising a non- pathogenic virus effective to inhibit the infectious disease in said animal.
109. The method of embodiment 108, wherein the non-pathogenic virus is an insect-specific virus.
110. The method of embodiment 109, wherein the insect-specific virus is a virus of Baculaviridae family.
111. The method of embodiment 110, wherein the virus of Baculaviridae family is a granulosis virus or a nucleopolyhedrosis virus.
112. The method of embodiment 111, wherein the nucleopolyhedrosis virus is Autographa californica nucleopolyhedrosis virus.
113. The method of embodiment 108, wherein said non-pathogenic virus is an inactivated virus, a viral particle, a virosome, a Virus Like Particle, a viral occlusion body, or a viral component.
114. A pharmaceutical composition comprising a non-pathogenic virus and a pharmaceutically acceptable carrier.
115. The pharmaceutical composition of embodiment 114 wherein said non- pathogenic virus is an inactive virus.
116. The pharmaceutical composition of embodiment 114 wherein said non- pathogenic virus is an insect-specific virus.
117. The pharmaceutical composition of embodiment 116 wherein said insect- specific virus is a virus of Baculaviridae family.
118. The pharmaceutical composition of embodiment 117, wherein the virus of Baculaviridae family is a granulosis virus or a nucleopolyhedrosis virus.
119. The pharmaceutical composition of embodiment 118, wherein the nucleopolyhedrosis virus is Autographa califomica nucleopolyhedrosis virus.
120. The pharmaceutical composition of embodiment 114 wherein said non- pathogenic virus is an inactive virus.
121. The pharmaceutical composition of embodiment 120 wherein said inactivation was one or more of genetic inactivation, chemical inactivation, photochemical inactivation, UV-light inactivation, heat inactivation, or radiological inactivation.
122. The pharmaceutical composition of embodiment 121 wherein said inactivation was at least two of genetic inactivation, chemical inactivation, photochemical inactivation, UV-light inactivation, heat inactivation, or radiological inactivation.
123. The pharmaceutical composition of embodiment 121 wherein said genetic inactivation is a temperature sensitive mutation.
124. A pharmaceutical composition comprising a non-pathogenic virus inactivated by at least two different methods.
125. The pharmaceutical composition of embodiment 124, wherein said virus was inactivated by at least three different methods.
126. A pharmaceutical composition comprising a non-pathogenic virus and a carrier wherein said carrier is a liposome, a virus like particle, a virosome or a protein delivery vehicle.
127. The pharmaceutical composition of embodiment 126 wherein said virus is inactive.
128. The pharmaceutical composition of embodiment 124, wherein said inactivation is least two different methods selected from the group consisting of genetic inactivation, chemical inactivation, photochemical inactivation, UV-light inactivation, heat inactivation, or radiological inactivation.
129. The pharmaceutical composition of embodiment 125, wherein said inactivation is at least three different methods selected from the group consisting of genetic inactivation, chemical inactivation, photochemical inactivation, UV-light inactivation, heat inactivation, or radiological inactivation.
130. A pharmaceutical composition comprising a non-pathogenic virus, said non-pathogenic virus inactivated using two or more methods selected from the group consisting of genetic inactivation, chemical inactivation, photochemical inactivation, UV-light inactivation, heat inactivation, or radiological inactivation, and at least one PBMC.
131. The pharmaceutical composition of embodiment 130 further comprising at least one cancer cell.
132. A process for preparing an anti-cancer or anti-infectious disease composition comprising a non-pathogenic virus, said process comprising: (a) exposing the virus to a first inactivator effective to inactivate an active virus; (b) exposing said virus to a second inactivator effective to inactivate an active virus; (c) combining said virus with one or more pharmaceutically acceptable carriers or excipients; and (d) confirming inactivity of said virus in an in vitro assay.
133. The process of embodiment 132 further comprising collecting a random portion of said anti-cancer composition for analysis of one or more of safety, efficacy, or toxicity.
134. The process of embodiment 133 wherein said first inactivator is genetic inactivation, chemical inactivation, photochemical inactivation, UV-light inactivation, heat inactivation, or radiological inactivation.
135. The process of embodiment 133 wherein said second inactivator is genetic inactivation, chemical inactivation, photochemical inactivation, UV-light inactivation, heat inactivation, or radiological inactivation, with the proviso that the first inativator is not the same as the second inactivator.
136. The process of embodiment 133 further comprising comparing one or more of safety, efficacy, or toxicity of said random portion of said anti-cancer or anti-infectious disease composition to historical data of safety, efficacy, or toxicity of a second anti-cancer composition.
137. The process of embodiment 132 wherein the inactivity of the virus is confirmed by plaque formation assay.
138. The process of embodiment 137 wherein said plaque formation assay is performed with Sf9 cells.
139. The process of embodiment 132 further comprising counting non-pathogenic virus using EM.
140. The process of embodiment 132 wherein confirmation of the inactivity of said virus is performed after each of (a) and (b).
141. The process of embodiment 136 wherein the results of said comparison are required to be compiled prior to the release of said an anti-cancer or anti-infectious disease composition.
142. The method of any one of embodiments 1,12, 31,35, 50,54, 58,62, 66,71, 76,84, 91, or 108 wherein said non-pathogenic virus is formulated as a pharmaceutical composition.
143. A pharmaceutical composition comprising an inactivated non- pathogenic virus and at least one antigen, wherein the antigen is distinct from the inactivated non-pathogenic virus, and at least one adjuvant.
144. An immunostimulating composition comprising an adjuvant composition, said adjuvant composition comprising an inactivated non-pathogenic virus, at least one antigen, wherein said antigen is distinct from the adjuvant composition, and further wherein said immunostimulating composition is capable of increasing the immune response to the antigen.
145. The immunostimulating composition of embodiment 144 wherein said inactivated non-pathogenic virus comprises a viral particle, a virosome, a Virus Like Particle, a viral occlusion body, or a viral component.
146. The method of embodiment 18 wherein said cancer cell is an epithelial cancer cell.
147. A pharmaceutical composition comprising a non-pathogenic virus and a peripheral blood mononuclear cells (PBMCs).
148. A method of treating cancer comprising administering the pharmaceutical composition of embodiment 147 to an individual in need thereof.

## Claims

1. Composition comprising a non-pathogenic, insect-specific virus for use in a method of treating or preventing an infectious disease in a subject.

2. Composition of claim 1, wherein said infectious disease that can be treated or prevented is a viral infection.

3. Composition of claim 2, wherein said viral infection is caused by a virus selected from the group of HIV, West Nile virus, hepatitis A, B, C, small pox, Vesicular Stomatitis Virus (VSV), Respiratory Syncytial Virus (RSV), human papilloma virus (HPV), influenza, Ebola, viral meningitis and herpes.

4. Composition of claim 3, wherein said wherein said viral infection is caused by Vesicular Stomatitis Virus (VSV).

5. Composition of claims 1, wherein the insect-specific virus is a virus of the family of Baculoviridae.

6. Composition of claim 5, wherein the Baculaviridae virus is a granulosis virus or a nucleopolyhedrosis virus.

7. Composition of claim 6, wherein the nucleopolyhedrosis virus is *Autographa californica* nucleopolyhedrosis virus.

8. Composition of claim 1, wherein said subject is a human.

9. Composition of claim 1, wherein the non-pathogenic, insect-specific virus is an inactivated virus, a viral particle, a virosome, a Virus-like particle, a viral occlusion body, or a viral component.

10. Composition of claim 9, wherein the viral component comprises at least two viral proteins.

11. Composition of claim 1, wherein the non-pathogenic, insect-specific virus is an inactivated virus.

12. Composition of claim 11, wherein the inactivation has been achieved by heat-inactivation, chemical inactivation, or UV inactivation, or a combination thereof.
